(19) **Europäisches Patentamt**

**European Patent Office**

**Office européen des brevets**

(11) **EP 1 514 945 A1**

(12) **EUROPEAN PATENT APPLICATION**

(43) Date of publication:
**16.03.2005 Bulletin 2005/11**

(51) Int Cl.[7]: **C12Q 1/68**, G01N 33/68, C07K 14/705

(21) Application number: **03292218.9**

(22) Date of filing: **09.09.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Warner-Lambert Company LLC Morris Plains, New Jersey 07950 (US)**

(72) Inventors:
• **Allen, Janet London EC1V 3RF (GB)**

• **Malderez-Bloes, Carole, c/o Pfizer PGRD 94265 Fresnes Cedex (FR)**
• **Schindler, Véronique, c/o Pfizer PGRD 94265 Fresnes Cedex (FR)**
• **Grentzmann, Guido, c/o Pfizer PGRD 94265 Fresnes Cedex (FR)**

(74) Representative:
**Dufresne, Guillaume Alain François et al PFIZER PGRD European Pharma Patent Dept. 23/25 Avenue du Docteur Lannelongue 95668 Paris Cedex 14 (FR)**

(54) **CD38 as a molecular marker on macrophages for COPD, and as a target molecule for treatment of COPD**

(57)     The present invention relates to use of CD38 as a biomarker for COPD. The invention also relates to methods of using compounds acting as CD38 modulators in the prevention and treatment of COPD. In addition, the invention provides methods and kits of diagnosis, prognosis, monitoring and assessment of the efficacy of a treatment for COPD using quantification of CD38 expression and/or activity levels.

EP 1 514 945 A1

**Description**

**Field of the invention** The present invention is in the field of molecular biology and diagnosis. In particular, the invention relates to CD38 as a target in COPD. CD38 may be useful in the prevention and treatment of this disease. In addition, macrophagic CD38 is a biomarker newly found to be associated with COPD. Such a biomarker may be used in the diagnosis, prognosis, and monitoring of COPD. Such biomarker may also be useful to assess the efficacy of a treatment for COPD as well as in screening assay to assess the efficiency of an agent to modulate CD38 expression.

**Background of the invention**

Macrophages can be segregated into two broad groups: resident tissue macrophages and recruited macrophages. Alveolar macrophages are heterogeneous, and those isolated from different anatomical sites, such as liver or kidney, differ in function presumably because of adaptive responses to the local micro-environment. Recruited macrophages are derived largely from circulating monocytes, which infiltrate damaged tissue, but some arise by local cell division. There is now increasing evidence for the heterogeneity of macrophages that have infiltrated inflamed or otherwise damaged tissue, depending on the type and severity of injury, the stage of its evolution and the localization of the macrophages within the tissue.
Macrophages are known to play a central role in the immune response and in inflammation.
They are also involved in the development of diseases such as autoimmune disorders and inflammatory diseases.
Many clinically significant disorders are indeed accompanied by inflammation, e.g. arthritis, bacterial infections, hypersensitivity, wound, cancer, etc.
Chronic obstructive pulmonary disease (COPD) is a chronic, slowly progressive disorder characterized by airflow obstruction (associated with airways inflammation). COPD include emphysema, chronic bronchitis, chronic airflow limitation, chronic airways obstruction, non-reversible obstructive airways disease, chronic obstructive airways disease and chronic obstructive lung disease. The clinical presentation of COPD can vary in severity from simple chronic bronchitis without disability to a severely disabled state with chronic respiratory failure. The diagnosis of COPD is usually suggested by symptoms but can only be established by quantitative measurements, preferably using spirometry (see Thorax, 1997; 52 suppl 5: S1-S32), hereby incorporated by reference in its entirety.
The Global Initiative for Chronic Obstructive Lung Disease recently issued guidelines (Pauwels *et al.* (2001) Resp. Care 46(8):798-825), hereby incorporated by reference in its entirety that proposed a four-stage classification of COPD severity, namely:

> Stage 0: At Risk- Characterized by chronic cough and sputum production. Lung function, as measured by spirometry, is still normal.
> Stage I: Mild COPD-Characterized by mild airflow limitation ($FEV_1$/FVC < 70% but $FEV_1$ > 80% predicted) and usually, but not always, by chronic cough and sputum production.
> Stage II-Moderate COPD: Characterized by worsening airflow limitation (30% < $FEV_1$ < 80% predicted) and usually the progression of symptoms with shortness of breath typically developing on exertion. The division into stages IIA (50% < $FEV_1$ < 80% predicted) and IIB (30% < $FEV_1$ < 50% predicted ) is based on the fact that exacerbations are especially seen in patients with a $FEV_1$ below 50% predicted.
> Stage III-Severe COPD: Characterized by severe airflow limitation with either; $FEV_1$ < 30% predicted or $FEV_1$ < 50% and the presence of respiratory failure or clinical signs of right heart failure.

FVC is the forced vital capacity and FEV1 the forced expiratory volume in one second as measured by spirometry.
Pulmonary emphysema is a major component of the morbidity and mortality of COPD, a condition that afflicts more than 14 million persons in the United States and has become the fourth leading cause of death. In healthy nonsmoker, macrophages comprise the major host cell defense cell in the lower airspace. Cigarette smoking is associated with a more than fivefold increase in total cells recovered by bronchoalveolar lavage (BAL), with macrophages comprising 95-98%. Moreover, macrophages are prominent in the respiratory bronchioles of cigarette smokers, where emphysematous changes are first manifest. (see Grashoff *et al.*, Am. J. Pathol. (1997);151(6):1785-90 and Shapiro (1999) Am. J. Respir. Crit. Care Med;160(5 Pt 2):S29-32).
In Eur. Respir. J., (1999); 14: 245-248, Haslam *et al.* suggest that the investigatory technique of bronchoalveolar lavage (BAL) has become one of the most valuable research tools for studying inflammatory mechanism in a wide range of diseases that affect the lung and airways in humans and that cytological and microbiological testing of BAL samples are of established value for assisting in clinical diagnosis and management of many lung diseases. These procedures are routinely available in most modem specialist respiratory centers.
There is a dearth of available information and extremely low public awareness about COPD. There are few efficacious alternative treatments available. Lack of awareness and the insidious nature of the disease have been major contributing factors to the low diagnostic rates seen in COPD. As the symptoms of the disease begin to occur with the onset of middle age, many patients dismiss symptoms such as brethlessness upon exertion as simply old age. Smokers, who constitute the majority of COPD sufferers, also commonly dismiss symptoms such as chronic cough as something to be expected as a result of smoking, rather than an indication of a serious underlying problem.

Diagnostic and treatment of COPD may be difficult to perform just according to disorder-related symptoms. Providing tools and methods allowing establishing a more precise diagnostic and treatment are then very useful.

There is then a real need to find biomarker of COPD that can be used for the different purposes including diagnostic and treatment of COPD. To our knowledge, only one study has shown that hyaluronate concentration in tracheal lavage fluid may be used as a marker of chronic inflammatory changes in the COPD-affected lung of horses (Tulamo and Maisi, (1997) Am. J. Vet. Res., 58, 729-732), but no biomarker of COPD exists for humans. The results obtained using this biomarker may be used for diagnostic purposes, alone or in combination with results obtained using other diagnostic methods in order to get a better diagnostic. Such biomarker may also be useful for prognosis, monitoring, assessing the efficacy of treatment of COPD as well as for preventing and treating this disease.

CD38 was initially defined by Reinherz and Schlossman in 1980 in their pioneering studies of thymocyte and T lymphocyte differentiation antigens (Reinherz at al, Proc Natl Acad Sci USA 1980;77:1588-92). The expression of CD38 was described as dependent on the stage of differentiation of the lymphocytes (reviewed in Malavasi et al. (1992), Int. J. Clin. Lab. Res. 22, 73-80 and Mehta et al.(1996), FASEB 10, 1408-1417.

Later on, CD38 was also identified in other hematopoïetic cells (macrophages, monocytes, NK cells, red blood cells, platelets) (Zocchi et al, (1993), Biochem. Biophys. Res. Comm. 196, 1459-1465 ; Ebihara et al, (1997) J. Biol. Chem. 272, 25, 16023-16029) as well as in non-hematopoietic cells (β-pancreatic cells, neurons) (Koguma et al, (1994), Biochim. Biophys. Acta 1223, 160-162). CD38 is not only present on the surface of the cell membrane but has also been shown to be associated to endoplasmic reticulum, mitochondria and nuclear membrane (Yamada et al, (1997), Brain Res. 756, 52-60). CD38 is also widely distributed in different tissues : pancreas (Takasawa et al, (1993a), Science, 259, 370-373), heart, thyroid gland, brain (Mizuguchi et al, (1995), Brain Research, 697, 235-240) (Yamada et al, (1997), Brain Res. 756, 52-60), eyes (Khoo et Chang, (1999), Brain Res. 821, 17-25) and other human tissues (Meszaros et al, (1995), Biochem. Biophys. Res. Commun. 210, 452-456).

A cDNA for human CD38 was isolated in 1990, this paved the way for the cloning of the human gene in 1997 (Jackson and Bell, (1990), J. Immunol. 144, 2811-2815). Initially defined in human, CD38 has also been cloned from mice and rat (Harada et al, (1993), J. Immunol. 151, 3111-3118 ; Koguma et al, (1994), Biochim. Biophys. Acta, 1223, 160-162).

The CD38 gene spans over 62 kilobases and consists of eight exons and seven introns of heterogenous length (Ferrero and Malavasi (1997), J. Immunol. 159, 3858-3865). The gene coding for CD38 was assigned to chromosome 4 (Katz et al, (1983), Eur. J. Immunol., 13, 1008-1013).

There has been little insight into the molecular mechanisms underlying regulation of CD38 expression. So far only the all-trans retinoic acid-induced CD38 expression (Kontani et al., (1993), J. Biol. Chem., 268, 16895-16898), which involves direct binding of a retinoic acid receptor transcription factor to a consensus sequence in intron 1 of the CD38 gene has been studied in detail (Kishimoto et al., (1998), J. Biol. Chem., 273, 15429-15434 ; Mills et al, (1998), Br. J. Haematol., 103, 87-92).

The human protein encoded by the CD38 gene is a type II single chain transmembrane glycoprotein of 45 kD. This protein is highly conserved in different species and is constituted of 300 residues. The architecture of the molecule consists of three regions : intracellular (21 amino acids), transmembranar (21 amino acids) and extracellullar (256 amino acids) (Kyte and Doolittle (1982) J. Mol. Biol., 157, 1, 105-132 ; Eisenberg (1984) Annu Rev Biochem, 53, 595-623)

In contrary to the two other regions, the extracellular region presents high structural homologies with CD38 from other species (mice, rat) and also with a soluble ADP-ribosyl cyclase purified from the mollusc Aplysia californica with which it presents 69% of sequence homology and 30% identity (States et al, (1992), Trends Biochem Sci., 17, 495).

This finding of a structural and also functional similarity with this enzyme marked the turning point in the study of the molecule and biochemists focused their attention on the catalytic activities of CD38 (Daeglio et al, (2001), Leukemia Research, 25, 1-12).

The first evidence for the enzymatic activity of CD38 was reported in the murine model in 1993 (Howard et al, (1993), Science, 262, 1056-1059) and was later confirmed in humans (Gelman et al, (1993), 23, 3361-3364). The extracellular part of CD38 has been expressed and presents an ADP-ribosyl cyclase and cADPR hydrolase (Howard et al, (1993), Science, 262, 1056-1059). CD38 expressed in COS-7 cells (Takasawa et al, (1993) J. Biol. Chem., 268, 26052-26054), and native human CD38 purified from red blood cells present also these activities (Zocchi et al, (1993) Biochem. Biophys. Res. Comm., 196, 1459-1465). Therefore CD38 is a multifunctional ectoenzyme which catalytic domain is at the C terminal facing the outside of the cell, and which controls four different catalytic activities :

1. The main activity of CD38 consists in its convertion of NAD+ to ADPR (NAD+glycohydrolase activity); or
2. it also converts NAD+ to cADPR (a second messenger that regulates the mobilisation of intracellular Ca++ ions) (ADP-ribosyl cyclase activity); and

3. cADPR to ADPR (cADPR hydrolase activity) (Howard et al, (1993), Science, 262, 1056-1059) (Deterre et al, (1996), J. Immunol., 157, 1381-1388).

4. In addition to generating ADPR and cADPR, CD38 may function as a mono-ADP-ribosyl transferase (Grimaldi et al, (1995), J. Immunol., 155, 811-817). Recombinant murine CD38 was shown to elicit auto-ribosylation and to ribosylate a number of different proteins at their cysteine residues.

CD38 is also involved in the complex pathways that rule cell growth and communication (Malawasi et al, (1994), Immunol. Today, 15, 95-98 ; Mehta et al, (1996), FASEB J., 10, 1408-1416). Direct ligation of human and murine CD38 by means of agonistic monoclonal antibodies triggers in vitro activation and proliferation of T, B, myeloid and NK cells (Funaro et al, (1990), J. Immunol., 145, 1390-1395), induces cytokine production (Ausiello et al, (1995), Eur. J. Immunol., 25, 1477-1482) and elicits phosphorylation of discrete cytoplasmic proteins (Zubiaur et al, (1997), J. Immunol., 159, 193-205 ; Silvennoinen et al, 1996, J. Immunol., 156, 100-107). CD38 is involved in the regulation of adhesion between cells (Dianzani et al, (1994), J. Immunol., 153, 952-959) and is physically and functionnally associated to other cell surface receptors, specialized in the transduction of intracellular signals, like CD3/TCR, BCR/CD19/CD21 or CD16 on T, B, and NK cells respectively (Funaro et al, (1993), Eur. J. Immunol., 23, 2407-2411).

A CD38 ligand was identified by means of its monoclonal antibody Moon-1 mAb. The molecule was identified as CD31, a well known member of the immunoglobulin (Ig) superfamily and a molecule deeply involved in human epithelial cells/ lymphocyte adhesion processes (Newman, (1999), 103, 5-9).

The CD38/CD31 interactions have been analyzed : in the normal or tumoral T cells, the signalisation obtained following the activation of CD38 with an agonist antibody, i.e. intracellular Ca2+ variation and induction of the cytokines production, is also obtained by induction of interactions between CD38 and CD31 (Deaglio et al, (1998), J. Immunol., 160, 395-402.

CD38 has been associated with different diseases, like leukemia (Hamblin et al, (1999), Blood, 94, 1848-1854) myeloma, solid tumors (Kramer et al, (1995), J. Urol. 154, 1636-1641), Burton agammaglobulinemia (Santos-Argumedo et al, (1995), Intern. Immunol., 7, 163-170), HIV infections of cells (Vigano et al, (2000), Chem. Immunol., 75, 207-211), type II diabetes (Okamoto et al, (1997), Diabetologia, 40, 1485-1491), Alzheimer disease (Otsuka et al, (1994), Brain Pathol., 4, 558) asthma (Fernandez et al, (1998), J. Biol. Reg. And Homeost. Agents, 12, 81-91) and active sarcoidosis (Ivna Svoboda Beusan et al, (1998), Period. Biol., 100, 483-487)

While the vast body of data on CD38 is derived from studies carried out with lymphocytes, very little is known about the function and regulation of CD38 present on monocytes/macrophages (Pfister et al, (2001), Eur. J. Biochem. 268, 5601-5608).

Circulating monocytes bear the molecule on their surface, (Deaglio et al, (2001), Leukemia research 25, 1-12). It has been shown that differenciation of monocytes to macrophages resulted in down-regulation of surface expression of CD38. This decrease correlates with a reduction in NAD+glycohydrolase activity, indicating that the amount of functional active CD38 molecules decreases during differentiation (Pfister et al, (2001), Eur. J. Biochem. 268, 5601-5608). CD38 has also been detected in residential macrophages, like in alveolar macrophages (Zocchi et al, (1993), Biochem. Biophys. Res. Comm., 196, 1459, 1465; Ebihara et al, (1997), J. Biol. Chem., 272, 16023-16029).

It must be mentioned that besides the very interesting function in immune system activation, it has been shown that glucose-induced insulin secretion is enhanced in transgenic mice that overproduce CD38 and attenuated in CD38 KO mice (Mallone et al, (2001), Diabetis 50, 752). Therefore, it is recommended to monitor the glycaemia of patients who overexpress CD38 is recommended.

**Summary of the invention**

<u>a) Uses of biomarkers</u>

The invention relates to the use of CD38 as a macrophagic biomarker of COPD. In a first embodiment, it relates to the use of CD38 in the *in vitro* diagnosis, prognosis or monitoring of COPD.

In a second embodiment, it relates to the use of CD38 in assessing the efficacy of treatment for COPD.

In a third embodiment, it relates to the use of CD38 in any of the methods and kits disclosed herein.

In a fourth embodiment, said use comprises the use of a CD38 polynucleotide, a sequence complementary thereto, or a fragment thereof in a hybridization or amplification assay and/or the use of compounds able to bind to a CD38 polynucleotide, a sequence complementary thereto, or a fragment thereof.

In a fifth embodiment, said use comprises the use of a CD38 polypeptide or a fragment thereof, and/or the use of compounds able to bind to a CD38 polypeptide or a fragment thereof in protein quantifying assays.

b) Diagnostic, prognosis and monitoring methods for COPD using quantification of CD38 expression and/or activity in macrophages.

Other objects of the invention are method of diagnosis, prognosis and monitoring of COPD for an individual, wherein said method comprises the step of quantifying the level of macrophagic CD38 expression and/or activity levels in at least one biological sample recovered from said individual using any one of the quantifying methods described herein.

In a first embodiment, said method is a method of determining whether an individual suffers from COPD, wherein said method comprises the steps of

a) quantifying the macrophagic level of CD38 expression and/or activity in a biological sample recovered from said individual using any one of the quantifying methods described herein; and

b) determining whether said macrophagic level of CD38 expression and/or activity are elevated in said individual compared to the macrophagic level of CD38 expression and/or activity in a control sample, wherein said elevated level of macrophagic CD38 expression and/or activity is taken as a sign of the presence of COPD.

In a further embodiment, said control sample originates from an individual not suffering from COPD. In an even further embodiment, said individual not suffering from COPD is a healthy individual. In another further embodiment, said individual not suffering from COPD is said individual before he suffers from COPD.

In a second embodiment, said method is a method of determining whether an individual who already suffers from a disorder different from COPD is susceptible to develop COPD, wherein said method comprises the steps of

a) quantifying the macrophagic level of CD38 expression and/or activity levels in a biological sample recovered from said individual using any one of the quantifying methods described herein; and

b) determining whether said macrophagic level of CD38 expression and/or activity in said individual is similar to the macrophagic CD38 expression and/or activity level of an individual suffering from COPD,

wherein said similar level of macrophagic CD38 expression and/or activity is taken as a sign of the evolution of COPD.

In a third embodiment, said method is a method of determining whether an individual who already suffers from COPD is susceptible to move into a more advanced stage of COPD, wherein said method comprises the steps of

a) quantifying the macrophagic level of CD38 expression and/or activity in a biological sample recovered from said individual using any one of the quantifying methods described herein; and

b) determining whether said macrophagic level of CD38 expression and/or activity in said individual is similar to the macrophagic level of CD38 expression and/or activity of an individual suffering from a more advanced stage of COPD;

wherein said similar level of macrophagic CD38 expression and/or activity is taken as a sign of the evolution for said individual towards said more advanced stage of COPD.

In a further embodiment, said individual who already suffers from chronic obstructive pulmonary disease is at stage 0 and said individual suffering from a more advanced stage of chronic obstructive pulmonary disease is at stage I. In another even further embodiment, said individual who already suffers from chronic obstructive pulmonary disease is at stage I and said individual suffering from a more advanced stage of chronic obstructive pulmonary disease is at stage II. In another even further embodiment, said individual who already suffers from chronic obstructive pulmonary disease is at stage II and said individual suffering from a more advanced stage of chronic obstructive pulmonary disease is at stage III.

In a fourth embodiment, said method is a method of monitoring an individual suffering from COPD, wherein said method comprises the steps of

a) quantifying the macrophagic level of CD38 expression and/or activity in different samples recovered from said individual at different time points; and

b) comparing the macrophagic levels of CD38 expression and/or activity in said different samples.

In a further embodiment, said different time points correspond to different stages of COPD. In another further embodiment, said method comprises the additional step of determining whether macrophagic levels of CD38 expression and/or activity increases over time, wherein said increase is taken as a sign of an increased degree of severity of COPD .

Still another object of the invention is a method of assessing the efficacy of a treatment for an individual suffering from COPD and submitted to a treatment, wherein said method comprises the steps of

a) quantifying the macrophagic levels of CD38 expression and/or activity in a biological sample recovered from said individual using any one of the quantifying methods described herein; and
b) determining whether said macrophagic level of CD38 expression and/or activity is modulated in said biological sample compared to the macrophagic level of CD38 expression and/or activity in a control sample.

In a further embodiment, said method is a method of assessing the efficacy of a treatment for an individual suffering from COPD, wherein said method comprises the steps of

a) quantifying the macrophagic levels of CD38 expression and/or activity in a biological sample recovered from said individual using any one of the quantifying methods described herein; and
b) determining whether said macrophagic levels of CD38 expression and/or activity in said individual is decreased compared to the macrophagic level of CD38 expression and/or activity in an individual suffering from COPD but not submitted to said treatment,

wherein a decreased level of said macrophagic CD38 expression and/or activity is taken as a sign of the efficacy of said treatment.

In any of the methods cited above, said biological sample is a sample recovered from any one of blood, broncho alveolar lavage, sputum, hematopoeitic tissues including but not limited to lung.

In any of the methods cited above, said biological sample comprises inflammatory, infiltrating recruited macrophages and/or tissue resident macrophages, preferably inflammatory macrophages from bronchio-pulmonary tissues.

c) Kits

Another aspect of the invention is to provide kits for performing methods according to the invention. Therefore another object of the invention is a kit, preferably a diagnostic kit for detection of COPD, wherein said kit comprises reagents to determine the level of CD38 expression and/or activity.
In a first embodiment, the invention relates to kits, preferably diagnostic kits for COPD, containing necessary material to quantify the amount of CD38 polynucleotides in a biological sample.
In a further embodiment, said kit comprises at least one oligonucleotide able to hybridize to a CD38 polynucleotide, a sequence complementary thereto or a fragment thereof. In an even further embodiment, said oligonucleotide is a nucleic acid probe able to hybridize specifically to said CD38 polynucleotide, sequence complementary thereto or fragment thereof. In a preferred embodiment, said kit comprises amplification primers, preferably primers of SEQ ID No 1 and/or SEQ ID No 2, able to amplify a region of a CD38 polynucleotide, preferably using real-time PCR.
In a second embodiment, the invention also relates to kits, preferably diagnostic kits for COPD, containing necessary material to quantify the amount of CD38 polypeptides in a biological sample.
In a further embodiment, said kit comprises an anti-CD38 antibody able to bind specifically to a CD38 polypeptide or fragment thereof. In an even further embodiment, said anti-CD38 antibody is labeled. In another even further embodiment, said kit comprises necessary reagent to perform an ELISA. In still another even further embodiment, said kit further comprises a reagent allowing the detection of the antigen-antibody complexes formed, said reagent optionally carrying a label, or being able to be recognized itself by a label reagent, particularly in the case when the above mentioned anti-CD38 antibody is not itself labeled. In a third embodiment, the invention also relates to kits, preferably diagnostic kits for COPD, containing necessary material to quantify the amount of CD38 activity in a biological sample. In a preferred embodiment, said kit comprises necessary reagents to measure CD38 enzymatic activities. Examples of CD38 substrates include NAD+, cADPR and its nonhydrolysable analogue 3-deaza-cADPR. In another further preferred embodiment, said kit comprises necessary reagents to measure NAD+ glycohydrolase and/or ribosyl-cyclase activities.

d) use of CD38 modulators for the preparation of a medicament for the prevention or the treatment of COPD.

Another object of the invention relates to the use of a CD38 modulator, preferentially a CD38 inhibitor, for the preparation of a medicament for the prevention or the treatment of COPD.
Another object of the invention is a method for the prevention or the treatment of COPD comprising administering to a mammal in need thereof, an effective amount of a CD38 modulator.
In a first embodiment, said compound is able to inhibit or reduce CD38 expression and/or activity.
In a second embodiment, said compound is able to reduce CD38 expression and/or activity.
In a third embodiment, said compound is able to bind to a CD38 polynucleotide or a fragment thereof. In a further embodiment, said compound is able to bind to regulatory sequences of said CD38 polynucleotide or fragment thereof. In another further embodiment, said compound is an antisense oligonucleotide or an oligonucleotide able to form a triple helix with said polynucleotide or fragment thereof.

In a fourth embodiment, said compound is able to bind to a CD38 polypeptide or a fragment thereof. In a further embodiment, said compound able to bind to said CD38 polypeptide or fragment thereof is an anti-CD38 antibody or a fragment thereof. In another further embodiment, said compound able to bind to said CD38 polypeptide or fragment thereof is a CD38 antagonist or a fragment thereof.

In a fifth embodiment, said compound is a non functional CD38 polypeptide or a fragment thereof.

In a sixth embodiment, said compound is a CD38 inhibitor selected from the group consisting of nicotinamide 2'-deoxyriboside, 5-methylnicotinamide 2'-deoxyriboside, sodium nitroprusside and N-ethylmaleimide, or a combination thereof.

**Detailed description of the invention**

I. Definition of terms

As used herein, the term « macrophage » encompasses any type of macrophages including recruited macrophages, tissue resident macrophages, or a mixture of recruited macrophages and tissue resident macrophages, irrespective of their tissular origin. Preferably, this term refers to recruited macrophages. More preferably, this term refers to recruited macrophages from bronchio-pulmonary origin such as BAL, lung tissue or sputum.

Recruited macrophages

The term "recruited macrophages", as used herein, relates to macrophages that are recruited to tissues by constitutive or inflammatory recruitment. They infiltrate damaged tissue, but some arise by local cell division. They are derived largely from circulating blood monocytes.

Tissue resident macrophages

The term "tissue resident macrophages", as used herein, refers to macrophages that are present in tissues. They are derived largely from circulating blood monocytes. There is now increasing evidence for the heterogeneity of macrophages that have infiltrated inflamed or otherwise damaged tissue, depending on the type and severity of injury, the stage of its evolution and the localization of the macrophages within the tissue. They can exhibit wide variation in morphology in tissues and undertake a wide range of physiological functions. Alveolar macrophages are heterogeneous, and those isolated from different anatomical sites, such as liver or kidney, differ in function presumably because of adaptive responses to the local micro-environment; Kupffer cells that line the liver sinusoids are important for the uptake and clearance of modified LDL and bacterial endotoxins via scavenger receptors such as SR-A, while macrophages in granulomatous lesions kill bacteria by generating reactive oxygen intermediates and nitric oxide.

Biomarker

The term "biomarker", as used herein refers to a biological macromolecule which expression and/or activity level is correlated with a biological phenomenon. Preferably, this term usually refers to a gene which expression and/or activity level increases by at least approximately 1.7 fold to 1000-fold, preferably 2-fold to 50-fold, more preferably 2-fold, 5-fold, 10-fold, 15-fold, 20-fold, 30-fold, 50-fold, 100-fold, 200-fold, 500-fold, 1000-fold, or more in a biological sample compared to a control sample.

CD38

The term « CD38 », as used herein, refers to CD38 expression products, i.e. CD38 polynucleotides and polypeptides, and fragments thereof.

CD38 polypeptides

The term "CD38 polypeptide or CD38 protein", when used herein, encompasses all CD38 polypeptides, preferably from mammalian species, more preferably from human and murine species, as well as their variants, analogs, orthologs, homologs, and derivatives, and fragments thereof that are characterized by their ability to exhibit a CD38 biological activity :

    1. the main activity of CD38 consists in its convertion of NAD+ to ADPR (NAD+glycohydrolase activity); or

2. it also converts NAD+ to cADPR (a second messenger that regulates the mobilisation of intracellular Ca++ ions) (ADP-ribosyl cyclase activity); and

3. cADPR to ADPR (cADPR hydrolase activity) (Howard et al, (1993), Science, 262, 1056-1059) (Deterre et al, (1996), J. Immunol., 157, 1381-1388).

4. In addition to generating ADPR and cADPR, CD38 may function as a mono-ADP-ribosyl transferase (Grimaldi et al, (1995), J. Immunol., 155, 811-817). Recombinant murine CD38 was shown to elicit auto-ribosylation and to ribosylate a number of different proteins at their cysteine residues.

Illustrating but not limiting examples of amino acid sequences of such CD38 polynucleotides may be found in Kyte and Doolittle, (1982) J Mol Biol., 157(1):105-32 and Eisenberg, 1984, Annu Rev Biochem, 53, 595-623), and also in sequence databases such as Genseq, Swissprot, Genbank, Embl, and PIR. Preferably, the term "CD38 polypeptide or protein" refers to the CD38 polypeptides as well as their variants, homologs and derivatives exhibiting essentially the same CD38 biological activity. By a polypeptide exhibiting essentially the same CD38 biological activity, it is intended that such a polypeptide exhibits 60% activity, preferably 70%, more preferably 80% of CD38 activity.

The term "CD38 polypeptide variants", as used herein, refers to polypeptides from the same species but differing from a reference CD38 polypeptide. Generally, differences are limited so that the amino acid sequences of the reference and the variant are closely similar overall and, in many regions, identical. Preferably, CD38 polypeptides are at least 70%, 80%, 90%, 95%, 96%, 97%, 98% or 99% identical to a reference CD38 polypeptide. By a polypeptide having an amino acid sequence at least, for example, 95% "identical" to a query amino acid sequence, it is intended that the amino acid sequence of the subject polypeptide is identical to the query sequence except that the subject polypeptide sequence may include up to five amino acid alterations per each 100 amino acids of the query amino acid sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence. The query sequence may be an entire amino acid sequence of the reference sequence or any fragment specified as described herein.

Such CD38 polypeptide variants may be naturally occurring variants, such as naturally occurring allelic variants encoded by one of several alternate forms of a gene occupying a given locus on a chromosome of an organism, or isoforms encoded by naturally occurring splice variants originating from a single primary transcript. Alternatively, a CD38 polypeptide variant may be a variant that is not known to occur naturally and that can be made using art-known mutagenesis techniques.

It is known in the art that one or more amino acids may be deleted from the N-terminus or C-terminus of a bioactive peptide or protein without substantial loss of biological function (see for instance, Ron *et al.*, (1993), Biol Chem., 268 2984-2988 ; which disclosure is hereby incorporated by reference in its entirety).

It also will be recognized by one of ordinary skill in the art that some amino acid sequences of CD38 polypeptides can be varied without significant effect of the structure or function of the protein. Such mutants include deletions, insertions, inversions, repeats, and substitutions selected according to general rules known in the art so as to have little effect on activity. For example, guidance concerning how to make phenotypically silent amino acid substitutions is provided in Bowie *et al.* (1990), Science 247:1306-1310, hereby incorporated by reference in its entirety, wherein the authors indicate that there are two main approaches for studying the tolerance of an amino acid sequence to change.

The first method relies on the process of evolution, in which mutations are either accepted or rejected by natural selection. The second approach uses genetic engineering to introduce amino acid changes at specific positions of a cloned gene and selections or screens to identify sequences that maintain functionality. These studies have revealed that proteins are surprisingly tolerant of amino acid substitutions. The authors further indicate which amino acid changes are likely to be permissive at a certain position of the protein. For example, most buried amino acid residues require nonpolar side chains, whereas few features of surface side chains are generally conserved. Other such phenotypically silent substitutions are described in Bowie *et al.*, (1990) supra, and the references cited therein.

Typically seen as conservative substitutions are the replacements, one for another, among the aliphatic amino acids Ala, Val, Leu and Phe; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr. In addition, the following groups of amino acids generally represent equivalent changes: (1) Ala, Pro, Gly, Glu, Asp, Gln, Asn, Ser, Thr; (2) Cys, Ser, Tyr, Thr; (3) Val, Ile, Leu, Met, Ala, Phe; (4) Lys, Arg, His; (5) Phe, Tyr, Trp, His.

The term CD38 polypeptide also encompasses all CD38 polypeptides encoded by CD38 gene analogs, orthologs, and/ or species homologues. As used herein, the term "gene analogs" refers to genes of different and unrelated organisms which perform the same functions in each organism but which did not originate from an ancestral structure that the organisms' ancestors had in common. Instead, analogous genes arose separately and then later evolved to perform the same function (or similar functions). In other words, analogous CD38 polypeptides are polypeptides with quite different amino acid sequences but that perform the same biological activity, namely CD38 biological activity. As used herein, the term "gene orthologs" refers to genes within two different species which sequences are related to each other via a common homologous gene in an ancestral species but which have evolved to become different from each other. As used herein, the term "gene homologs" refers to genes of different organisms which perform the same functions in each organism and which originate from an ancestral structure that the organisms' ancestors had in common. In other words, homologous CD38 polypeptides are polypeptides with quite similar amino acid sequences that perform the same biological activity, namely CD38 biological activity. Preferably, CD38 polypeptide homologs may be defined as polypeptides exhibiting at least 40%, 50%, 60%, 70%, 80%, 90%, 95%, 96%, 97%, 98% or 99% identity to a reference CD38 polypeptide. As used herein, the term "CD38 polypeptide derivatives" refers to modified CD38 polypeptides during or after translation, e.g., by glycosylation, acetylation, phosphorylation, amidation, derivatization by known protecting/blocking groups, proteolytic cleavage, linkage to an antibody molecule or other cellular ligand, and the like. Any of numerous chemical modifications may be carried out by known techniques, including but not limited, to specific chemical cleavage by cyanogen bromide, trypsin, chymotrypsin, papain, V8 protease, $NaBH_4$; acetylation, formylation, oxidation, reduction; and metabolic synthesis in the presence of tunicamycin.

Additional post-translational modifications encompassed by the invention include, for example, e.g., N-linked or O-linked carbohydrate chains, processing of N-terminal or C-terminal ends, attachment of chemical moieties to the amino acid backbone, chemical modifications of N-linked or O-linked carbohydrate chains, and addition or deletion of an N-terminal methionine residue as a result of prokaryotic host cell expression. The polypeptides may also be modified with a detectable label, such as an enzymatic, fluorescent, isotopic or affinity label to allow for detection and isolation of the protein.

Also provided by the invention are chemically modified derivatives of CD38 polypeptides that may provide additional advantages such as increased solubility, stability and circulating time of the polypeptide, or decreased immunogenicity. See U.S. Patent No: 4,179,337, hereby incorporated by reference in its entirety. The chemical moieties for derivatization may be selected from water soluble polymers such as polyethylene glycol, ethylene glycol/propylene glycol copolymers, carboxymethylcellulose, dextran, polyvinyl alcohol and the like. The polypeptides may be modified at random positions within the molecule, or at predetermined positions within the molecule and may include one, two, three or more attached chemical moieties.

Thus, a CD38 polypeptide according to the invention may be, for example: (i) one in which one or more of the amino acid residues are substituted with a conserved or non-conserved amino acid residue (preferably a conserved amino acid residue) and such substituted amino acid residue may or may not be one encoded by the genetic code: or (ii) one in which one or more of the amino acid residues includes a substituent group: or (iii) one in which the CD38 polypeptide is fused with another compound, such as a compound to increase the half-life of the polypeptide (for example, polyethylene glycol): or (iv) one in which the additional amino acids are fused to the above form of the polypeptide, such as an IgG Fc fusion region peptide or leader or secretory sequence or a sequence which is employed for purification of the above form of the polypeptide or a pro-protein sequence.

CD38 polypeptides may be monomers or multimers. Multimers may be dimers, trimers, tetramers or multimers comprising at least five monomeric polypeptide units. Multimers may also be homodimers or heterodimers. Multimers of the invention may be the result of hydrophobic, hydrophilic, ionic and/or covalent associations and/or may be indirectly linked, by for example, liposome formation. In one example, covalent associations are between the heterologous sequences contained in a fusion protein containing a CD38 polypeptide or fragment thereof (see, e.g., US Patent Number 5,478,925, which disclosure is hereby incorporated by reference in its entirety). In another example, a CD38 polypeptide or fragment thereof is joined to one or more polypeptides that may be either CD38 polypeptides or heterologous polypeptides through peptide linkers such as those described in U.S. Pat. No. 5,073,627 (hereby incorporated by reference). Another method for preparing multimer CD38 polypeptides involves use of CD38 polypeptides fused to a leucine zipper or isoleucine zipper polypeptide sequence known to promote multimerization of the proteins in which they are found using techniques known to those skilled in the art including the teachings of WO 94/10308. In another example, CD38 polypeptides may be associated by interactions between Flag® polypeptide sequence contained in fusion CD38 polypeptides containing Flag® polypeptide sequence. CD38 multimers may also be generated using chemical techniques known in the art such as cross-linking using linker molecules and linker molecule length optimization techniques known in the art (see, e.g., US 5,478,925), techniques known in the art to form one or more inter-molecule cross-links between the cysteine residues located within the sequence of the polypeptides desired to be contained in the multimer (see, e.g., US 5,478,925), addition of cysteine or biotin to the C terminus or N-terminus of CD38 polypeptide and techniques to generate multimers containing one or more of these modified polypeptides (see, e.g., US 5,478,925), or any of the 30 techniques to generate liposomes containing CD38 multimers (see, e.g., US Patent Number 5,478,925,), which disclosures are incorporated by reference in their entireties.

As used herein, the term "CD38 polypeptide fragment" refers to any peptide or polypeptide comprising a contiguous span of a part of the amino acid sequence of a CD38 polypeptide.

More specifically, a CD38 polypeptide fragment comprising at least 6, preferably at least 8 to 10, more preferably 12, 15, 20, 25, 30, 35, 40, 50, 60, 75, 100, 125, 150, 175, 200, 225, 250, 275, or 300 consecutive amino acids of a CD38 polypeptide according to the present invention. CD38 polypeptide fragment may additionally be described as sub-genuses of CD38 polypeptides comprising at least 6 amino acids, wherein "at least 6" is defined as any integer between 6 and the integer representing the C-terminal amino acid of a CD38 polypeptide. Further included are species of CD38 polypeptide fragments at least 6 amino acids in length, as described above, that are further specified in terms of their N-terminal and C-terminal positions. Also encompassed by the term "CD38 polypeptide fragment" as individual species are all CD38 polypeptide fragments, at least 6 amino acids in length, as described above, that may be particularly specified by a N-terminal and C-terminal position. That is, every combination of a N-terminal and C-terminal position that a fragment at least 6 contiguous amino acid residues in length could occupy, on any given amino acid sequence of the present invention is included in the present invention.

It is noted that the above species of polypeptide fragments of the present invention may alternatively be described by the formula "a to b"; where "a" equals the N-terminal most amino acid position and "b" equals the C-terminal most amino acid position of the polynucleotide; and further where "a" equals an integer between 1 and the number of amino acids of a CD38 polypeptide sequence minus 6, and where "b" equals an integer between 7 and the number of amino acids of the CD38 polypeptide sequence; and where "a" is an integer smaller then "b" by at least 6.

The above CD38 polypeptide fragments can be immediately envisaged using the above description and are therefore not individually listed solely for the purpose of not unnecessarily lengthening the specification. Moreover, the above fragments do not necessarily need to have a CD38 biological activity, although polypeptides having these activities are preferred embodiments of the invention, since they would be useful, for example, in immunoassays, in epitope mapping, epitope tagging, as vaccines, and as molecular weight markers. The above fragments may also be used to generate antibodies to a particular portion of the polypeptide.

Also encompassed by the term "CD38 polypeptide fragment" are domains of CD38 polypeptides. Such domains may eventually comprise linear or structural motifs and signatures including, but not limited to, leucine zippers, helix-turn-helix motifs, post-translational modification sites such as glycosylation sites, ubiquitination sites, alpha helices, and beta sheets, signal sequences encoding signal peptides which direct the secretion of the encoded proteins, sequences implicated in transcription regulation such as homeoboxes, acidic stretches, enzymatic active sites, substrate binding sites, and enzymatic cleavage sites. Such domains may present a particular biological activity such as DNA or RNA-binding, secretion of proteins, transcription regulation, enzymatic activity, substrate binding activity, etc...

A domain has a size generally comprised between 3 and 1000 amino acids. In preferred embodiment, domains comprise a number of amino acids that is any integer between 6 and 200. Domains may be synthesized using any methods known to those skilled in the art, including those disclosed herein for the preparation of CD38 polypeptides to produce anti-CD38 antibodies. Methods for determining the amino acids that make up a domain with a particular biological activity include mutagenesis studies and assays to determine the biological activity to be tested. Indeed, it has been shown that the extracellular part of CD38 presents an ADP-ribosyl cyclase and cADPR hydrolase activity (Howard et al, (1993), Science, 262, 1056-1059). Critical amino acids for CD38 enzymatic activity have been described in (Tohgo et al., (1994) *J. Biol. Chem.* 269(46):28555-7): Mutation of C 119 to K, or C201 to E induces a loss of cADPR hydrolase activity ; Mutation of C119 to R,E,A, or C160 to A, or C173 to A, or C201 to D,K,A induce a loss of cADPR hydrolase and ADP-ribosyl cyclase activity.

Alternatively, the polypeptides of the invention may be scanned for motifs, domains and/or signatures in databases using any computer method known to those skilled in the art. Searchable databases include Prosite (Hofmann *et al.,* (1999) *Nucl. Acids Res.* 27:215-219; Bucher and Bairoch (1994) Proceedings 2nd International Conference on Intelligent Systems for Molecular Biology. Altman *et al*, Eds., pp53-61, AAAIPress, Menlo Park), Pfam (Sonnhammer *et al.,* (1997) *Proteins* 28(3):405-20; Henikoff *et al.,* (2000) *Nucleic Acids Res.* 28(1):228-30; Bateman *et al.,*(2000) *Nucleic Acids Res.* 28(1):263-6), Blocks (Henikoff *et al.,*(2000) *Electrophoresis* 21(9):1700-6), Print (Attwood *et al.,* (1996) *Nucleic Acids Res.* 24(1):182-8, Prodom (Sonnhammer and Kahn (1994) *Protein Sci.* 3(3):482-92; Corpet *et al.* (2000) *Nucleic Acids Res.* 28(1):267-9), Sbase (Pongor *et al.* (1993) *Protein Eng.* 6(4):391-5; Murvai *et al.,* (2000) *Nucleic Acids Res.* 28(1):260-2), Smart (Schultz *et al.,* (1998) *Proc. Natl. Acad. Sci. U S A* 95, 5857-5864), Dali/FSSP (Holm and Sander (1996) *Nucleic Acids Res.* 24(1):206-9; Holm and Sander (1997) *Nucleic Acids Res.* 25(1):231-4; Holm and Sander (1999) *Nucleic Acids Res.* 27(1):244-7), HSSP (Sander and Schneider (1991) *Proteins* 9(1):56-68.), CATH (Orengo *et al.,* (1997) *Structure* 5(8):1093-108; Pearl *et al.,* (2000) *Biochem. Soc. Trans.* 28(2):269-75), SCOP (Murzin *et al.,* (1995) *J. Mol. Biol.* 247(4):536-40; Lo Conte *et al.,* (2000) *Nucleic Acids Res.* 28(1):257-9), COG (Tatusov *et al.,* (1997) *Science* 278, 631 :637 ; Tatusov *et al.,* (2000) *Nucleic Acids Res.* 28(1):33-6), specific family databases and derivatives thereof (Nevill-Manning *et al.,* (1998) *Proc. Natl. Acad. Sci. U S A.* 95, 5865-5871; Yona *et al.,* (1999) *Proteins* 37(3):360-78; Attwood *et al.,* (2000) *Nucleic Acids Res.* 28(1):225-7), each of which disclosures are hereby incorporated by reference in their entireties. For a review on available databases, see issue 1 of volume 28 of *Nucleic Acid Research* (2000), which disclosure is hereby incorporated by reference in its entirety.

The term "CD38 polypeptide fragment" also encompasses epitopes-bearing fragments. These epitopes may be antigenic epitopes or both an antigenic epitope and an immunogenic epitope. An immunogenic epitope is defined as a part of a protein that elicits an antibody *response in vivo* when the polypeptide is the immunogen. On the other hand, a region of polypeptide to which an antibody binds is defined as an antigenic epitope. An epitope can comprise as few as 3 amino acids in a spatial conformation, which is unique to the epitope. Generally an epitope consists of at least 6 such amino acids, and more often at least 8-10 such amino acids.

A CD38 epitope-bearing fragment according to the invention may be any fragment which length is between 6 amino acid and the full-length sequence of a CD38 polypeptide, preferably a fragment between 6 and 50 amino acid. The epitope-bearing fragments may be specified by either the number of contiguous amino acid residues (as a sub-genus) or by specific N-terminal and C-terminal positions (as species) as described above.

Fragments which function as epitopes may be produced by any conventional means *(See, e.g.*, Houghten (1985), Proc. Natl. Acad. Sci. USA 82:5131-5135 and U.S. 4,631,21, which disclosures are hereby incorporated by reference in their entireties). Methods for determining the amino acids which make up an epitope include x-ray crystallography, 2-dimensional nuclear magnetic resonance, and epitope mapping, e.g., the Pepscan method described by Geysen *et al.*, (1984), Proc. Natl. Acad. Sci. U.S.A. 81:3998-4002; PCT Publications WO 84/03564 and WO 84/03506, which disclosures are hereby incorporated by reference in their entireties. Another example is the algorithm of Jameson and Wolf, (1988), Comp. Appl. Biosci. 4:181-186 (said reference incorporated by reference in its entirety). The Jameson-Wolf antigenic analysis, for example, may be performed using the computer program PROTEAN, using default parameters (Version 4.0 Windows, DNASTAR, Inc.)

The present invention also provides for the exclusion of any CD38 fragment species specified by N-terminal and C-terminal positions or of any fragment sub-genus specified by size in amino acid residues as described above. Any number of fragments specified by N-terminal and C-terminal positions or by size in amino acid residues as described above may be excluded as individual species. The present invention also provides for the exclusion of any CD38 domain or epitope-bearing fragment in the same manner.

The CD38 polypeptides of the present invention can be prepared in any suitable manner. Such CD38 polypeptides and fragments thereof may be purified from natural sources, chemically synthesized, produced by recombinant techniques including *in vitro* translation techniques or expression in a recombinant cell able to express CD38 cDNA, or a combination of these methods, using techniques known to those skilled in the art (See, for example, "Methods in Enzymology, Academic Press, 1993" for a variety of methods for purifying proteins; Creighton, (1983) Proteins: Structures and Molecular Principles, W.H. Freeman & Co. 2nd Ed., T. E., New York; and Hunkapiller *et al.*, (1984) Nature. 310(5973): 105-11 for chemical synthesis of proteins and Davis *et al.* (1986) Basic Methods in Molecular Biology, ed., Elsevier Press, NY for recombinant techniques, which disclosures are incorporated by reference in their entireties). The polypeptides of the present invention are preferably provided in an isolated form, and may be partially or preferably substantially purified.

CD38 activity

The term « CD38 biological activity » and « CD38 activity » refer to all activities exhibited by CD38 polypeptides including but not limited to NAD+glycohydrolase activity, ADP-ribosyl-cyclase activity, cADPR hydrolase activity and mono-ADP-ribosyl transferase. The term « CD38 activity » also encompasses any biological activity induced by CD38 such as regulation of the mobilisation of intracellular Ca++ ions, modulation of the cytokine production, tyrosine phosphorylation of proteins, modulation of activation and cellular proliferation, modulation of the transduction of intracellular signals, like CD3/TCR, BCR/CD19/CD21 or CD 16 on T, B, and NK cells respectively, human immunodeficiency virus infection proliferation, lymphopoiesis, apoptosis, adhesion.

CD38 activities may be measured using any assay known to those skilled in the art including those described elsewhere in the application.

CD38 polynucleotides

The term "CD38 polynucleotide", when used herein, encompasses all polynucleotides, including genomic DNA, mRNA and cDNA nucleic acid molecules, encoding a CD38 polypeptide as defined below, preferably from mammalian species, more preferably from human and murine species, as well as their variants, analogs, orthologs, homologs, and derivatives, and fragments thereof. Illustrating but not limiting examples of nucleic acid sequences of such CD38 polynucleotides may be found in sequence databases such as Genbank, EMBL, OMIM, Genseq, RefSeqn, Unigene or in the following publications: (Kyte and Doolittle (1982) J. Mol. Biol., 157, 1, 105-132; Eisenberg (1984) Annu Rev Biochem, 53, 595-623).

In one preferred embodiment, the term "CD38 polynucleotide" refers to the polynucleotides encoding the CD38 polypeptides as well as its variants and homologs exhibiting essentially the same CD38 biological activity. As used herein, the term "CD38 polynucleotides variant" refers to polynucleotides from the same species but differing from a reference polynucleotide. Generally, differences are limited so that the nucleotide sequences of the reference and the variant are closely similar overall and, in many regions, identical. Preferably, CD38 polynucleotides are at least 50%. 60%, 70%, 80%, 90%, 95%, 96%, 97%, 98% or 99% identity to a reference CD38 polynucleotide. By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the CD38 polypeptide. The query sequence may be an entire nucleic sequence of the reference polynucleotides, or the ORF (open reading frame) of the reference polynucleotide, or any fragment specified as described herein. Such CD38 polynucleotide variants may be naturally occurring variants, such as naturally occurring allelic variants being one of several alternate forms of a gene occupying a given locus on a chromosome of an organism, or naturally occurring splice variants originating from a single primary transcript. Alternatively, a CD38 polynucleotide variant may be a variant that is not known to occur naturally and that can be made using art-known mutagenesis techniques.

Such CD38 polynucleotides variants may be degenerate variants, i.e. polynucleotides that comprise a sequence substantially different from those described above but which, due to the degeneracy of the genetic code, still encode a CD38 polypeptide according to the present invention. That is, all possible polynucleotide sequences that encode the CD38 polypeptides of the present invention are completed. This includes the genetic code and species-specific codon preferences known in the art. Thus, it would be routine for one skilled in the art to generate the degenerate variants described above, for instance, to optimize codon expression for a particular host (e.g., change codons in the human mRNA to those preferred by other mammalian or bacterial host cells).

Nucleotide changes present in a variant polynucleotide may be silent, which means that they do not alter the amino acids encoded by the polynucleotide. However, nucleotide changes may also result in amino acid substitutions, additions, deletions, fusions and truncations in the polypeptide encoded by the reference sequence. The substitutions, deletions or additions may involve one or more nucleotides. The variants may be altered in coding or non-coding regions or both. Alterations in the coding regions may produce conservative or non-conservative amino acid substitutions, deletions or additions. In the context of the present invention, preferred embodiments are those in which the polynucleotide variants encode CD38 polypeptides that retain substantially the same biological properties or activities as the CD38 protein. More preferred polynucleotide variants are those containing conservative substitutions.

The term CD38 polynucleotides also encompasses CD38 gene analogs, orthologs, and/or species homologues as defined above. Preferably, CD38 polynucleotide homologs may be defined as polynucleotides exhibiting at least 50%, 60%, 70%, 80%, 90%, 95%, 96%, 97%, 98% or 99% identity to a reference CD38 polynucleotide.

As used herein, the term "CD38 polynucleotide fragment" refers to any oligonucleotide or polynucleotide comprising a contiguous span of a part of the nucleic acid sequence of a CD38 polynucleotide.

More specifically, a CD38 polynucleotide fragment comprising at least 8, preferably at least 10, 12, 15, or 18, more preferably at least 20, 25, 28, 30, 35, 40, 50, 75, 100, 150, 200, 300, 400, 500, 800, or 1000 nucleotides of a CD38 polynucleotide according to the present invention. CD38 polynucleotide fragment may additionally be described as sub-genuses of CD38 polynucleotides comprise at least 8 nucleotides, wherein "at least 8" is defined as any integer between 8 and the integer representing the 3' most nucleotide position of a CD38 polynucleotide.

Further included are species of CD38 polynucleotide fragments at least 8 nucleotides in length, as described above, that are further specified in terms of their 5' most and 3' most positions. Also encompassed by the term "CD38 polynucleotide fragment" as individual species are all CD38 polynucleotide fragments, at least 8 nucleotides in length, as described above, that may be particularly specified by a 5' most and 3' most nucleotide position. That is, every combination of a 5' most and 3' most nucleotide position that a fragment at least 8 contiguous nucleotide residues in length could occupy, on any given nucleic acid sequence of the sequence listing or of the present invention is included in the present invention. The 5' and 3' positions are represented by the position numbers set forth in the appended sequence listing. For allelic, degenerate and other variants, position 1 is defined as the 5' most nucleotide of the ORF, i.e., the nucleotide "A" of the start codon with the remaining nucleotides numbered consecutively. Therefore, every combination of a 5' and 3' nucleotide position that a polynucleotide fragment of the present invention, at least 8 contiguous nucleotides in length, could occupy on a polynucleotide of the invention is included in the invention as an individual species. The above CD38 polynucleotide fragments can be immediately envisaged using the above description and are therefore not individually listed solely for the purpose of not unnecessarily lengthening the specification.

It is noted that the above species of CD38 polynucleotide fragments of the present invention may alternatively be described by the formula "a to b"; where "a" equals the 5' most nucleotide position and "b" equals the 3' most nucleotide position of the CD38 polynucleotide; and further where "a" equals an integer between 1 and the number of nucleotides of the polynucleotide sequence of the present invention minus 8, and where "b" equals an integer between 9 and the number of nucleotides of the polynucleotide sequence of the present invention; and where "a" is an integer smaller then "b" by at least 8.

Also encompassed by the term "CD38 polynucleotide fragment" are polynucleotides comprising polynucleotides encoding domains of CD38 polypeptides or polynucleotides comprising epitope-bearing fragments, as defined above.

The present invention also provides for the exclusion of any CD38 fragment species specified by 5' most and 3' most nucleotide or of any fragment sub-genus specified by size in nucleotide residues as described above. Any number of fragments specified by 5' most and 3' most nucleotide positions or by size in nucleotide residues as described above may be excluded as individual species. The present invention also provides for the exclusion of any polynucleotides fragment encoding a CD38 domain or epitope-bearing fragment in the same manner.

Identity Between Polynucleotides Or Polypeptides

The terms "polynucleotides having at least x% identity with a polynucleotide of reference" and "polypeptides having at least x% identity with a polypeptide of reference" encompass polynucleotides or polypeptides which residue (nucleotide or amino acid respectively) sequence exhibit an identity percentage, as defined below, equal or superior to x compared to said reference polynucleotide or polypeptide sequence, respectively.
The identity percentage is determined after optimal alignment of two polynucleotides or polypeptide sequences over a comparison window, wherein portions of the polynucleotide or polypeptide sequences in the comparison window may comprise additions or deletions of one or more residue in order to optimize sequence alignment. The comparison window contains a certain number of positions (either a residue or a gap corresponding to an insertion/deletion of a residue), this number of positions corresponding to the window size. Each window position may present one of the following situations:

1°/ There is a residue (nucleotide or amino acid) on this position on the first aligned sequence and a different residue at the same position on the second aligned sequence, in other words the second sequence has a substituted residue at this position compared to the first sequence.
2°/ There is a residue (nucleotide or amino acid) on this position on the first aligned sequence and the same residue at the same position on the second aligned sequence.
3°/ There is a residue (nucleotide or amino acid) on this position on the first aligned sequence and no residue at the same position on the second aligned sequence, in other words the second sequence presents a deletion at this position compared to the first sequence.

The number of positions within the comparison window belonging to the first above-defined category is called R1.
The number of positions within the comparison window belonging to the second above-defined category is called R2.
The number of positions within the comparison window belonging to the third above-defined category is called R3.
The identity percentage (%id) may be calculated by any of the following formulas:

$$\%id = R2/(R1+R2+R3) \times 100,$$

or

$$\%id = (R2+R3)/(R1+R2+R3) \times 100$$

Alignment of sequences to compare may be performed using any of the variety of sequence comparison algorithms and programs known in the art. Such algorithms and programs include, but are by no means limited to, TBLASTN, BLASTP, FASTA, TFASTA, , FASTDB, WU-BLAST, Gapped-BLAST, PSI-BLAST (Pearson and Lipman, (1988), Proc. Natl. Acad. Sci. USA 85:2444-2448; Altschul et al., (1990), J. Mol. Biol. 215:403-410; Altschul et al., (1993), Nature Genetics 3:266-272; Altschul et al., (1997), Nuc. Acids Res. 25:3389-3402; Thompson et al., (1994), Nuc. Acids Res.. 22:4673-4680; Higgins et al., (1996), Meth. Enzymol. 266:383-402; Brutlag et al. (1990) Comp. App. Biosci. 6:237-245; Jones and Swindells, (2002) Trends Biochem Sci 27:161-4; Olsen et al. (1999) Pac Symp Biocomput; 302-13), the disclosures of which are incorporated by reference in their entireties.
In a particular embodiment, the Smith-Waterman method is used with scoring matrix such as PAM, PAM 250 or preferably with BLOSUM matrices such as BLOSUM60 or BLOSUM62 and with default parameters (Gap Opening Penalty=10 and Gap Extension Penalty=1) or with user-specified parameters preferably superior to default parameters.
In another particular embodiment, protein and nucleic acid sequences are aligned using the Basic Local Alignment Search Tool ("BLAST") programs with the default parameters or with modified parameters provided by the user. Preferably, the scoring matrix used is the BLOSUM62 matrix (Gonnet et al., (1992), Science 256:1443-1445; Henikoff and Henikoff, (1993), Proteins 17:49-61, which disclosures are hereby incorporated by reference in their entireties). Less preferably, the PAM or PAM250 matrices may also be used (see, e.g., Schwartz and Dayhoff, (1978), eds., Matrices for Detecting Distance Relationships: Atlas of Protein Sequence and Structure, Washington: National Biomedical Research Foundation, which disclosure is hereby incorporated by reference in its entirety).

In still another particular embodiment, polynucleotide or polypeptide sequences are aligned using the FASTDB computer program based on the algorithm of Brutlag *et al.* (1990), *supra.* Preferred parameters used in a FASTDB alignment of DNA sequences are: Matrix=Unitary, k-tuple=4, Mismatch Penalty= 1, Joining Penalty=30, Randomization Group Length=0, Cutoff Score= 1, Gap Penalty=5, Gap Size Penalty 0.05, Window Size=500 or the length of the subject nucleotide sequence, whichever is shorter. Preferred parameters used in a FASTDB amino acid alignment are: Matrix=PAM 0, k-tuple=2, Mismatch Penalty= 1, Joining Penalty=20, Randomization Group25Length=0, Cutoff Score= 1, Window Size=sequence length, Gap Penalty=5, Gap Size Penalty=0.05, Window Size=500 or the length of the subject amino acid sequence, whichever is shorter.

Polynucleotide

As used interchangeably herein, the terms "nucleic acid molecule(s)", "oligonucleotide(s)", and "polynucleotide(s)" include RNA or DNA (either single or double stranded, coding, complementary or antisense), or RNA/DNA hybrid sequences of more than one nucleotide in either single chain or duplex form (although each of the above species may be particularly specified). The term "nucleotide" is used herein as an adjective to describe molecules comprising RNA, DNA, or RNA/DNA hybrid sequences of any length in single-stranded or duplex form. More precisely, the expression "nucleotide sequence" encompasses the nucleic material itself and is thus not restricted to the sequence information (i.e. the succession of letters chosen among the four base letters) that biochemically characterizes a specific DNA or RNA molecule.

The term "nucleotide" is also used herein as a noun to refer to individual nucleotides or varieties of nucleotides, meaning a molecule, or individual unit in a larger nucleic acid molecule, comprising a purine or pyrimidine, a ribose or deoxyribose sugar moiety, and a phosphate group, or phosphodiester linkage in the case of nucleotides within an oligonucleotide or polynucleotide. The term "nucleotide" is also used herein to encompass "modified nucleotides" which comprise at least one modifications such as (a) an alternative linking group, (b) an analogous form of purine, (c) an analogous form of pyrimidine, or (d) an analogous sugar. For examples of analogous linking groups, purine, pyrimidines, and sugars see for example PCT publication No. WO 95/04064, which disclosure is hereby incorporated by reference in its entirety. Polynucleotides of the invention, including CD38 polynucleotides or fragments thereof, can be obtained using methods that comprise sequentially linking together nucleotides to produce the nucleic acids having the desired sequences. Polynucleotides of the invention may be synthesized enzymatically using genetic engineering techniques well known to those skilled in the art including cloning and restriction of appropriate sequences, amplification-based methods, hybridization-based methods as described herein. Alternatively, a variety of chemical methods of synthesizing nucleic acids are known to those skilled in the art including but not limited to direct chemical synthesis by a method such as the phosphodiester method of Narang *et al.*, (1979) Methods Enzymol 68:90-98, the phosphodiester method of Brown *et al.*, (1979), *Meth. Enzymol.* 68:109-151, the diethylphosphoramidite method of Beaucage *et al.*, (1981) *Tetrahedron Lett*, 22: 1859-1862 and the solid support method described in EP 0 707 592, which disclosures are hereby incorporated by reference in their entireties. In many of these methods, synthesis is conducted on a solid support. These included the 3' phosphoramidite methods in which the 3' terminal base of the desired oligonucleotide is immobilized on an insoluble carrier. The nucleotide base to be added is blocked at the 5' hydroxyl and activated at the 3' hydroxyl so as to cause coupling with the immobilized nucleotide base. Deblocking of the new immobilized nucleotide compound and repetition of the cycle will produce the desired polynucleotide. Alternatively, polynucleotides may be prepared as described in U.S. Patent No. 5,049,656, which disclosure is hereby incorporated by reference in its entirety. In some embodiments, several polynucleotides prepared as described above are ligated together to generate longer polynucleotides having a desired sequence.

Anti-CD38 antibodies

As used herein, "anti-CD38 antibody" is meant to include whole antibodies, including single-chain whole antibodies, antigen binding fragments thereof, and any functional or recombinant derivatives thereof, which are capable of binding the whole CD38 polypeptide or a CD38 epitopic determinant. Antigen binding antibody fragments include, but are not limited to, Fab, Fab' F(ab)2 and F(ab')2, Fd, single-chain Fvs (scFv), single-chain antibodies, disulfide-linked Fvs (sdFv) and fragments comprising either a $V_L$ or $V_H$ domain. Antigen-binding antibody fragments, including single-chain antibodies, may comprise the variable region(s) alone or in combination with the entire or partial of the following: hinge region, CH1, CH2, and CH3 domains. Also encompassed by the anti-CD38 antibody term are any combinations of variable region(s) and hinge region, CH1, CH2, and CH3 domains. Such anti-CD38 antibody may be a hybrid, chimeric, humanized, univalent, monoclonal or polyclonal antibody. It may be monospecific, bispecific, trispecific or have greater multispecificity. Multispecific antibodies may be specific for different epitopes of a CD38 or may be specific for both a polypeptide of the present invention as well as for heterologous compositions, such as a heterologous polypeptide or solid support material. *See, e.g.,* WO 93/17715; WO 92/08802; WO 91/00360; WO 92/05793; Tutt, *et al.* (1991), J. Immunol. 147:60-69; US Patents 5,573,920, 4,474,893, 5,601,819, 4,714,681, 4,925,648; Kostelny *et al.* (1992), ), J. Immunol. 148:1547-1553, which disclosures are hereby incorporated by reference in their entireties.

## II. Uses of a biomarker

As described in the Example section, applicants have found that CD38 expression was shown to increase in BAL macrophages and in trachea of mice that were smoking during 10 weeks and in macrophages derived from human blood monocytes after LPS stimulation.

Therefore, the invention relates to the use of CD38 as a macrophagic biomarker for COPD, in any method, process, assays of any nature, whether experimental or not, using the fact that an increase in CD38 expression and/or activity level is indicative of COPD.

Such use encompasses the use of experimental tools derived from CD38 expression products (CD38 polynucleotides or polypeptides) or fragments thereof, such as primers and probes able to hybridize to CD38 polynucleotides or fragments thereof, compounds able to bind to CD38 polypeptides or fragments thereof, such as anti-CD38 antibodies, as assayable biomarkers for COPD. CD38 may be used as a biomarker for COPD alone or in combination with another marker.

Such a biomarker may be useful for a number of different purposes including diagnostic, prognostic and monitoring purposes.

Indeed, such biomarker may be used, alone or in combination with other markers, to determine whether a patient suffers from COPD. It may also be used to determine the risk for a patient to develop complications associated with COPD. It may be used to monitor the degree of severity or the evolution of COPD in a patient. In addition, such biomarker may also be used to monitor the effect of a treatment of COPD on gene expression.

## III. Methods of determination of whether CD38 expression and/or activity is modulated

To determine whether CD38 expression and/or activity is modulated in a biological sample, the CD38 expression and/or activity level is measured in said biological sample and compared to the CD38 expression and/or activity level in a control.

Quantification of CD38 expression level and/or activity in a biological sample may be achieved using methods for determining the amount of CD38 polynucleotides in a biological sample, methods for determining the amount of CD38 polypeptides in a biological sample and/or methods for determining the amoumt of CD38 activity in a biological sample, as will be described below.

Determination of whether a CD38 expression and/or activity level is modulated in a test sample may be achieved through relative comparison of levels of CD38 expression and/or activity between said test sample and a control sample. Examples of test and control samples include: macrophages from an individual suffering from COPD versus an individual not suffering from said disorder, etc. Said comparison may be conducted through parallel quantification of both test sample and control sample submitted to the same experimental conditions in order to be able to directly compare the signal intensity given by both samples. In such case, relative and not necessarily absolute amounts of CD38 expression and/or activity are determined.

Alternatively, determination of a modulated level of CD38 expression and/or activity in a test sample may be achieved through absolute quantification of the amount of CD38 expression and/or activity level. Such absolute quantification may be performed either through parallel quantification of a test sample and at least one control sample containing a known amount of CD38 polynucleotides or polypeptides and submitted to the same experimental conditions. Such control samples allows to plot a signal vs. amount curve allowing absolute quantification to be performed. Alternatively, quantification of CD38 expression and/or activity is performed and compared to existing calibration curves allowing absolute quantification to be determined. Then, the CD38 expression and/or activity level in the test sample is compared to a reference level of CD38 expression and/or activity. Such references levels may be those determined for a healthy individual for example.

For the purpose of the methods of the invention, a CD38 expression and/or activity level in a sample will be considered modulated if a significant increase or decrease in CD38 expression and/or activity exists for said sample compared to a control sample. Said significant increase or decrease depends on the reproducibility and sensitivity of the assay used. Therefore the threshold of said increase or decrease to consider that CD38 expression and/or level are modulated depends on the quantification assay that is performed. Generally, a threshold of at least 1,7-fold, preferably at least 2-fold, preferably using quantitative PCR, preferably real-time PCR, will be considered significant to determine that the CD38 expression and/or activity is modulated, preferably elevated.

A method of determining whether CD38 expression is modulated in macrophages, preferentially recruited macrophages, of a biological sample comprises the steps of

a) quantifying CD38 expression and/or activity level in macrophages of said biological sample; and

b) determining whether said macrophagic CD38 expression and/or activity level in said biological sample is elevated compared to the macrophagic CD38 expression and/or activity level in a control sample.

In a first embodiment, said control sample is a sample containing macrophages from a healthy individual.

In a second embodiment, said determining step comprises determining whether said CD38 expression and/or activity level in macrophages of said biological sample is elevated approximately from 1.7-fold to 1000-fold, preferably from 10-fold to 100 fold, more preferably from 2-fold to 50 fold compared to said macrophagic CD38 expression and/or activity in said control sample.

In a third embodiment, said quantifying step comprises quantifying CD38 polynucleotides present in macrophages of said biological sample. In a further embodiment, said quantifying step is performed using a hybridization-based assay. In another further embodiment, said quantifying step is performed using an amplification-based assay selected from the group consisting of PCR assays, competitive PCR assays, real-time PCR assays, branched DNA-based signal amplification assays, nucleic acid sequence based amplification assays (NASBA), and transcription mediated amplification (TMA). In a preferred embodiment, said amplification is performed using PCR. In a further preferred embodiment, said amplification-based assay is real-time PCR. In another further preferred embodiment, said PCR is performed using the amplification primers of SEQ ID No 1 and 2. In a fourth embodiment, said quantifying step comprises quantifying CD38 polypeptides present in macrophages of said biological sample. In a further embodiment, said quantifying step comprises the use of a compound able to bind to a CD38 polypeptide or a fragment thereof. In an even further embodiment, said compound is an antibody or fragment thereof that binds specifically to said CD38 polypeptide or fragment thereof. In another even further embodiment, said compound is a CD38 ligand, like CD31, or a fragment thereof able to bind to CD38.

In a fifth embodiment, said quantifying step comprises quantifying CD38 activity in macrophages of said biological sample. In a further embodiment, said CD38 activity is quantified by measuring NAD+ glycohydrolase or ribosyl-cyclase activities. In an even further embodiment, said measurement of NAD+ glycohydrolase or ribosyl-cyclase activities are quantified by measuring the hydrolysis of NAD+.

In a sixth embodiment, said biological sample comprises infiltrating and/or tissue-resident macrophages, preferably from bronchio-pulmonary origin.

Another method of determining whether CD38 expression is modulated in macrophages, preferentially in recruited macrophages , of a biological sample comprises the steps of

a) optionally, purifying macrophages from said biological sample;

b) extracting nucleic acids from said macrophages;

c) contacting said extracted nucleic acids from said macrophages from said biological sample with at least one nucleic acid probe under conditions allowing hybridization to occur between said at least one nucleic acid probe and CD38 polynucleotides present in said biological sample in order to form a complex;

d) quantifying the amount of hybridized CD38 polynucleotides in said biological sample; and

e) determining whether said amount of hybridized CD38 polynucleotides in said biological sample is elevated compared to the macrophagic CD38 expression level in a control sample.

In a first embodiment, said at least one nucleic acid probe is labeled with a detectable molecule. In a second embodiment, said at least one nucleic acid probe has been immobilized on a substrate. In a further embodiment, said at least one nucleic acid probe that has been immobilized is immobilized on an array. In a third embodiment, said at least one nucleic acid probe has a sequence comprised in a sequence complementary to a CD38 polynucleotide.

Another method of determining whether CD38 expression is modulated in macrophages, preferentially in recruited macrophages, of a biological sample comprises the steps of

a) optionally, purifying macrophages from said biological sample;

b) extracting nucleic acids from said macrophages;

c) contacting said nucleic acids from said macrophages from said biological sample with amplification reaction reagents comprising a pair of amplification primers located on either side of a selected CD38 region to be amplified;

d) performing an amplification reaction to synthesize CD38 amplicons containing said selected CD38 region;

e) quantifying the amount of said CD38 amplicons; and

f) determining whether said amount of CD38 amplicons in said biological sample is elevated compared to the macrophagic CD38 expression level in a control sample.

Optionally, when the selected CD38 polynucleotide to be amplified is RNA, reverse transcription, and optionally synthesis of a second cDNA strand, are performed prior to said contacting step.

In a first embodiment, said quantifying step comprises hybridization with a labeled probe having a sequence complementary to the CD38 amplicon. In a second embodiment, said quantifying step comprises using incorporated labeled dNTPs, preferably fluorescent dNTPs. In a third embodiment, said quantifying step comprises using a double-stranded DNA specific dye, preferably a fluorescent dye. In a fourth embodiment, said amplification reaction is performed using semi-quantitative PCR or quantitative PCR, preferably real-time PCR.

Another method of determining whether CD38 expression is modulated in macrophages, preferentially in recruited macrophages, of a biological sample comprises the steps of

a) optionally, purifying macrophages from said biological sample;
b) extracting proteins from said macrophages;
c) contacting said proteins from said macrophages of said biological sample with a compound able to bind to a CD38 polypeptide or a fragment thereof under conditions allowing said compound to bind to said CD38 polypeptide or said fragment thereof;
d) quantifying the amount of said CD38 polypeptides; and
e) determining whether said amount of CD38 polypeptides in said biological sample is elevated compared to the macrophagic CD38 expression level in a control sample.

In a first embodiment, said compound is CD38 ligand CD31, or a fragment thereof able to bind to CD38.

In a second embodiment, said compound is an antibody or a fragment thereof able to bind to said CD38 polypeptide or fragment thereof. In a further embodiment, said antibody or fragment thereof is labeled with a detectable molecule. In another further embodiment, said antibody has been immobilized on a substrate.

In a third embodiment, said quantifying step comprises using an assay selected from the group consisting of enzyme-linked immunosorbent assays, radioimmunoassays, immunoblotting assays, immunofluorescent assays, immunoprecipitation assays, chemiluminescent assays, and immunohistochemical assays.

In a preferred embodiment, said assay is an enzyme-linked immunosorbent assay. In a first embodiment of said preferred embodiment, said contacting step comprises contacting a solid support or matrix to which are bound antibodies or fragments thereof that recognize a CD38 polypeptide or fragment thereof with said biological sample under conditions allowing the formation of immobilized CD38-antibody complexes, and wherein said quantifying step comprises detecting said immobilized CD38-antibody complexes via a labeled detection molecule, preferably another antibody, that specifically binds to said CD38 polypeptide or fragment thereof in said immobilized CD38-antibody complexes.

In a second embodiment of said preferred embodiment, said contacting step comprises incubating said sample with a first antibody that specifically recognizes and binds to a CD38 polypeptide or fragment thereof under conditions that allow the formation of CD38-antibody complexes, and wherein said quantifying step comprises adding a second antibody that bind to said CD38-antibody complexes, wherein said second antibody is an antibody that binds to a different CD38 epitope or an antibody that bind to the first antibody already bound to said CD38 polypeptides or fragment thereof. Said second antibody may either be labeled or coupled to a solid support in order to provide for a selectable marker.

In a third embodiment of said preferred embodiment, said contacting step comprises incubating said sample with an excess of antibody that specifically recognizes and binds to a CD38 polypeptide or fragment thereof under conditions that allow the formation of CD38-antibody complexes, and wherein said quantifying step comprises adding a known amount of unsolubilized recombinant CD38 polypeptide or fragment thereof to determine the amount of remaining unbound antibody. Preferably, said recombinant CD38 polypeptide or fragment thereof is labeled.

Another method of determining whether CD38 expression is modulated in macrophages, preferentially in recruited macrophages, in a biological sample comprises the steps of

a) optionally, purifying macrophages from said biological sample;
b) contacting said sample with said CD38 substrate such as NAD+, cADPR or its nonhydrolysable analogue 3-deaza-cADPR, or with said CD38 ligand CD31;
c) quantifying the amount of CD38 activity; and
d) determining whether said CD38 activity in said biological sample is elevated compared to the macrophagic CD38 activity level in a control sample.

In one embodiment, said CD38 activity is assayed measuring NAD+ glycohydrolase or ribosyl-cyclase activities by a variety of techniques including but not limiting to measurement of the hydrolysis of NAD+.

1. Suitable biological samples

Biological samples for use in the methods of the present invention include, without any particular limitation, any sample that contains macrophages or macrophage-like cells.

Such suitable samples may be obtained from a cell, a tissue or an animal, for example, a mammal, particularly a human. Biological samples suitable for use in this method include but are not limited to i) *in vitro* cultured macrophagic cell lines such as differentiated U937, THP1, HL60, and Raw 264.7, ii) biological fluids such as blood, broncho alveolar lavage, and iii) tissue samples (e.g. biopsies) especially from lung. Cell cultures or cell extracts derived, for example, from tissue biopsies can also be used. Depending on the quantification assays to be performed, as can be readily envisioned by the man skilled in the art, suitable biological samples include but are not limited to cell lysates, intact cells, supernatants, or frozen tissue sections.

Biological samples may originate from individuals suffering from COPD. In such cases, control samples originate from individuals not suffering from this disorder, preferably healthy individuals. Biological samples may also originate from individuals suffering from COPD to which a treatment has been or is being given. Said individual are animals, preferably mammals, more preferably humans or rodents.

These biological samples are illustrating but not limiting examples and one skilled in the art could envision and prepare other types of biological samples in different conditions.

Preparation of biological samples

Prior to quantification of CD38 expression levels and/or activity, additional steps of preparation of suitable samples for conducting assays may be necessary, such as macrophage isolation and/or macrophage purification. The necessity of such additional steps could be easily envisioned by one skilled in the art depending on the type of biological sample used and of the type of assay to be performed to determine CD38 expression levels and/or activity.

If the biological sample is a sample recovered from an animal, isolation of macrophages may be necessary when quantification techniques require that macrophages need to be removed from their natural environment in order for the assay to be performed.

Alternatively, quantification of levels of CD38 expression and/or activity may be performed using *in situ* techniques or histochemical techniques not requiring macrophage isolation. Techniques of isolation of macrophages from biological fluids, tissue and immune response sites are well known to those skilled in the art including isolation techniques described in "Handel-Fernandez and Lopez, "Macrophages", Practical Approach series, Chap 1, pp 1-30, Ed Paulnock, Oxford University Press (2000), and described in Example 1 of the PCT publication WO9747325, which disclosures are incorporated by reference in their entireties.

Optionally, macrophages may need to be purified from other sample materials, mostly from other cells, using any techniques known to those skilled in the art including adherence-based methods, velocity sedimentation-based methods, elutriation and isopycnic sedimentation methods such as those described in Gessani *et al.*, "Macrophages", Practical Approach series, Chap 2, pp 31-60, Ed Paulnock, Oxford University Press (2000), hereby incorporated by reference in its entirety. Further purification of macrophages may be obtained using magnetic activated cell sorting (MACS) techniques or fluorescence activated cell sorting (FACS) techniques such as those described in Ecsedy *et al.*, J. Lpid Res. (1998) 39:2218-27; Baldus *et al.*, Histochem. J. (1998) 30:285-91; Adeleye *et al*, Hum. Reprod. (1996) 11:451-6; Davey *et al.*, Br J. Haematol. (2000) 111:934-42 and Johnston *et al.*, Ann. Neurol. (2001) 49:650-8, which disclosures are incorporated by reference in their entireties. These techniques are based on the use of antibodies able to bind to macrophage cell surface antigens such as CD68, CD11b, CD18, CD14, CD32, F4/80, or SR-A. More macrophage cell surface antigens may be found in Weir's handbook of experimental immunology (5th edn), chapters 174 and 175, Ed Herzenberg *et al*, Blackwell Scientific publications (1997), hereby incorporated by reference in its entirety. Alternatively, the natural auto fluorescence of resident alveolar macrophages (Viksman *et al.*, (1994) J Immunol Methods;172(1): 17-24) may be used, and eventually enhanced by a fluorescent dye such as PHK26-PCL, to isolate them from other cells as described in Maus *et al.*, (2001) Am. J. Physiol. Lung Cell. Mol. Physiol. 280:L58-L68, which disclosures are incorporated by reference in their entireties.

2. Quantification of CD38 expression levels

Quantification of CD38 expression levels may be achieved either by measuring the amount of CD38 polynucleotides present in a sample or the amount of CD38 polypeptides present in a sample.

2.1. Quantification of CD38 polynucleotides present in a sample

Quantification of CD38 polynucleotides, either at the cellular or tissue level, may be achieved using any techniques known to those skilled in the art including hybridization-based techniques as well as amplification-based techniques.

Suitable samples for conducting these assays may be any type of samples containing any type of nucleic acid molecules. Such samples may contain either single stranded or double stranded molecules or a mixture thereof. They may either contain RNA, preferably mRNA, or contain DNA, preferably cDNA, or a mixture thereof.

Suitable biological samples to conduct such methods are samples in which nucleic acid molecules are accessible, including crude cell extracts or nucleic acid preparations (either RNA preparation or cDNA preparation). Preferably, nucleic acid molecules are extracted and eventually purified from biological samples using any techniques of preparation of total RNA or polyA RNA known to those skilled in the art such as those described *in Current Protocols in Molecular Biology,* John Wiley & Sons, Inc. (1997) and Sambrook and Russsell, (2001), Molecular Cloning: A Laboratory Manual 3rd ed. Cold Spring Harbor Laboratory, Cold Spring Harbor, New York, which disclosures are incorporated by reference in their entireties.

When cDNA preparation is necessary, reverse transcription and eventually synthesis of a second cDNA strand are performed preliminary to actual quantification of CD38 polynucleotides. Techniques for generating appropriate oligonucleotide primers for reverse transcription and primer extension, for reverse transcribing a primer hybridized to mRNA to generate a first cDNA strand, and for extending a primer to make a second cDNA strand complementary to the first cDNA strand are well known to those skilled in the art including those described *in Current Protocols in Molecular Biology,* (1997) supra and Sambrook and Russell, (2001), supra.

2.1.1. Using hybridization-based techniques

In one embodiment of said method of determining the level of gene expression, said CD38 expression level is determined using hybridization-based techniques. Techniques used to quantify the expression levels of nucleic acids capable of hybridizing to a detectable probe include techniques well known to those skilled in the art such as Northern blotting, Southern blotting, *in situ* hybridization, dot blotting, slot blotting or Rnase protection assays (Parker and Barnes, (1999) Meth. Mol. Biol. 106:247-283; Hod (1992) Biotechniques 13:852-854; Saccomano *et al.* (1992) Biotechniques 13:846-850; Sambrook and Russell (2001) supra; which disclosures are incorporated by reference in their entireties). In such hybridization-based techniques, a nucleic acid sample is incubated in presence of a nucleic acid probe, generally an oligonucleotide probe, under conditions allowing hybridization to occur between the probe and CD38 polynucleotides present in the sample to form a complex. Design of appropriate probe is well known to those skilled in the art and described elsewhere in the application. Preferably, stringent conditions of hybridization are used in order to ensure that the nucleic acid probe hybridizes only to nucleic acids molecules having a high degree of homology to the probe so that it binds specifically to its CD38 polynucleotide target. Generally, stringent conditions are selected to be about 5°C to 20°C lower than the thermal melting point (Tm) for the probe sequence at a defined ionic strength and pH. The Tm is the temperature (under defined ionic strength and pH) at which 50% of the target sequence hybridizes to a perfectly matched probe.

For probes between 14 and 70 nucleotides in length the melting temperature (Tm) may be calculated using the formula: Tm=81.5+16.6(log (Na+))+0.41(fraction G+C)-(600/N) where N is the length of the probe.

If the hybridization is carried out in a solution containing formamide, the melting temperature may be calculated using the equation: Tm=81.5+16.6(log (Na+))+0.41(fraction G+C)-(0.63% formamide)-(600/N) where N is the length of the probe.

For example, the following stringent hybridization conditions are suitable for a probe of about 20 nucleotide in length. The hybridization step is realized at 65°C in the presence of 6 x SSC buffer, 5 x Denhardt's solution, 0,5% SDS and 100µg/ml of salmon sperm DNA and is followed by four washing steps :

- two washings during 5 min, preferably at 65°C in a 2 x SSC and 0.1%SDS buffer;
- one washing during 30 min, preferably at 65°C in a 2 x SSC and 0.1% SDS buffer,
- one washing during 10 min, preferably at 65°C in a 0.1 x SSC and 0.1%SDS buffer.

There is no need to say that the hybridization conditions described above have to be adapted according to the length of the desired nucleic acid, following techniques well known to the one skilled in the art. The suitable hybridization conditions may for example be adapted according to the teachings disclosed in the book of Hames and Higgins (1985) Nucleic Acid Hybridization: A Practical Approach. Hames and Higgins Ed., IRL Press, Oxford or using the disclosure of Sambrook *et al.*, (1989), supra.

Following hybridization and washing steps, detection is performed using standard direct or indirect detection techniques well known to those skilled in the art.

In such assays, either the probe (as described below) or the polynucleotides sample containing the CD38 target sequence may be immobilized on any convenient support.

2.1.2. Using amplification-based techniques

In a preferred embodiment of said method of determining the level of gene expression in a biological sample, said CD38 expression level is determined using amplification-based techniques.

Optionally, when the CD38 polynucleotide to be amplified is a RNA molecule, preliminary reverse transcription and eventually synthesis of a second cDNA strand may be necessary to provide a DNA template to be amplified.

Amplification techniques that can be used in the context of the present invention include, but are not limited to semi-quantitative or quantitative PCR, including competitive PCR, non-competitive PCR and real-time PCR, branched DNA (bDNA)-based signal amplification assays, nucleic acid sequence based amplification assays (NASBA), and transcription mediated amplification (TMA).

*2.1.2.1. Quantitative PCR*

Quantitative PCR technology is the preferred amplification technique used in the present invention as it allows either relative or absolute quantification of specific nucleic acid molecules in a sample. A variety of quantitative PCR techniques are familiar to those skilled in the art including non-competitive PCR, competitive PCR and real-time PCR assays. For a review of experimental designs of quantitative PCR technology, see Freeman *et al.*, Biotechniques (1999) 26:112-22; Bustin, J. Mol. Endocrinol. (2000) 25:169-93; Watzinger *et al.*, Nuc. Acids Res. (2001) 29:E52-2, Walker (2001) J. Biochem. Molec. Toxicol. 15:121-127; US 5,824,516 and EP publications EP1138783A2 and EP1138784 A2, which disclosures are incorporated by reference in their entireties. In each of these PCR procedures, PCR primers on either side of the nucleic acid sequences to be amplified are added to a suitably prepared nucleic acid sample along with dNTPs and a thermostable polymerase such as Taq polymerase, Pfu polymerase, Tth polymerase or Vent polymerase. The nucleic acid in the sample is denatured and the PCR primers are specifically hybridized to complementary nucleic acid sequences in the sample. The hybridized primers are extended. Thereafter, another cycle of denaturation, hybridization, and extension is initiated. The cycles are repeated multiple times to produce an amplified fragment containing the nucleic acid sequence between the primer sites. Amplicons are then analyzed using any conventional means known to those skilled in the art including but not limited to size-separation on a polyacrylamide or agarose gel, hybridization with a labeled probe, detection of incorporated labeled dNTPs, and detection of double stranded DNA using a double stranded DNA specific dye such as Sybr Green or ethidium bromide.

In competitive PCR, different amounts of the specific target sequence are co amplified together with a dilution series of an internal standard of a known copy number. The initial copy number of the target sequence is then extrapolated from the mixture containing an identical PCR product quantity of standard and target sequence at the so-called equivalence point (Zimmermann and Mannhalter, (1996) Bio-Techniques 21:280-279; Freeman *et al*, (1999) supra; Watzinger *et al.* (2001), supra, which disclosures are incorporated by reference in their entireties).

In non-competitive PCR, the same amount of the specific target sequence is co amplified together with a dilution series of an internal standard of a known copy number. The initial copy number of the target sequence at the equivalence point is then defined as the intersection between the plot of the log of standard signal vs. the log of input standard and the plot of the target signal vs. the log of input standard (Freeman *et al.* (1999), supra).

In real-time PCR, the formation of PCR products is monitored in each cycle of the PCR. The amplification is usually measured in thermocyclers that have additional devices for measuring fluorescence signals during or after the amplification reaction. Typical examples of these apparatus are the Roche Diagnostics LightCycler and the Perkin-Elmer ABI Prism 7700. In real-time PCR, the fluorescence signal is displayed immediately after each measurement, allowing amplification runs to be terminated or extended as appropriate. The amplification products may be detected using fluorescent dyes that bind to double-stranded DNA such as SYBG Green and ethidium bromide. Alternatively, amplicons may be detected by hybridization to a fluorescence-labeled probe that is specific to the target sequence, thus obviating the need for further confirmation of the analysis of the amplicon. Such fluorescence-labeled probes may be molecular beacons that only emit fluorescence signals when they are bound to the target nucleic acid, end-labeled fluorescent probes used alone or in combination such as allowing detection of a signal only when two independent probes hybridize to their correct target sequence, or hydrolysis probes that are hydrolyzed only when bound to their target sequence such as Taqman probes (PE applied Biosystems). For a review on the different types of fluorescence-labeled probes see Bustin (2000), *supra.* For each series of experiments, a signal threshold corresponding to the signal intensity for which all PCR reactions to be analyzed are in an exponential phase is determined. The number of cycles, so-called Ct, required to reach this threshold value is then determined for the target nucleic acid as well as for a reference nucleic acid such as a standard or housekeeping gene. The absolute or relative copy numbers of the target molecule can be determined on the basis of the Ct values obtained for the target nucleic acid and the reference nucleic acid (Gibson *et al.*, (1996) Genome Research 6:995-1001; Bieche *et al.*, (1999) Cancer Research 59:2759-2765; Bustin (2000), supra; Freman *et al.* (2000), supra; WO 97/46707; and WO 97/46714, which disclosures are incorporated by reference in their entireties). A detailed example of use of real time RT-PCR to quantify CD38 expression level is given in Example 1.

*2.1.2.2. Other quantitative or semi-quantitative amplification techniques*

The NASBA and TMA technologies are isothermal nucleic acid amplification assays that are virtually identical in principle and practice. These isothermal nucleic acid amplification assays use oligonucleotide probes, a reverse transcriptase, Rnase H, a DNA polymerase and an RNA polymerase to amplify a target sequence. An isothermal nucleic acid amplification assay, unlike PCR, is an isothermal amplification method, thus it does not cycle between high and low temperatures to facilitate target amplification. NASBA was described in Guatelli *et al.*(1990) Proc. Natl. Acad. Sci. USA., 35:273-286 and in Compton (1991) *Nature* 350:91-92 and used to amplify human immunodeficiency virus type (HIV-1) (Kievits *et al.*, (1991) J. Virol. Meth., 35: 273-286), which disclosures are incorporated by reference in their entireties. TMA was described in PCT Publication WO 9322461, hereby incorporated by reference in its entirety. In a NASBA assay, for example, the target RNA molecule is placed into solution with oligonucleotide primers and two polymerases. The temperature of the sample is raised to prepare the target molecules for amplification. The temperature is then lowered to permit the primer bind to the template. After binding the target, a DNA molecule complementary to the target sequence is synthesized using a reverse transcriptase, such as the AMV-reverse transcriptase. Subsequent to this synthesis, the RNA-DNA hybrid molecule is digested with RNase H, removing the RNA strand. A complementary strand to the single stranded DNA target sequence is synthesized through another round of DNA polymerization using a DNA polymerase enzyme. Following the synthesis of this double stranded DNA molecule, single stranded RNA is synthesized from the double stranded DNA template with T7 RNA polymerase. This step amplifies the original target sequence 100 to 1000-fold. Simultaneously, new double stranded DNA templates are being synthesized from the replicated single stranded RNA templates produced in the first round of amplification. From this series of reactions, the target sequence is amplified.

In a branched DNA assay (bDNA), amplification involves previously synthesizing a branched polymer single- stranded DNA probe, and hybridizing the branched polymer single-stranded DNA probe to a target gene to detect the target gene as described in Urdea *et al.*, (1991) Nucleic Acids Symp. Ser. 24:197-200, hereby incorporated by reference in its entirety. Such amplification assays were used to quantify viral load as described in Wilber (1997) Immunol. Invest. 26:9-13, hereby incorporated by reference in its entirety.

### 2.1.3. Primers and probes

*2.1.3.1. Design of primers and probes*

Any polynucleotide able to hybridize to CD38 polynucleotides may be used as a primer or probe according to the invention. Particularly preferred probes and primers include polynucleotides comprising a contiguous span of at least 12, 15, 18, 20, 25, 30, 35, 40, 50, 60, 70, 80, 90, 100, 150, 200, 500, or 1000 nucleotides of a CD38 polynucleotide or of a sequence fully complementary thereto.

A probe or a primer according to the invention has between 8 and 1000 nucleotides in length. The length of primers can range from 8, 10, 15, 20, 30, or 100 to 500 nucleotides, preferably from 10 to 50, more preferably from 15 to 30 nucleotides. The length of probes can range from 8, 10, 15, 20, 30, 50, 100, 200, 500, or 1000 nucleotides, preferably from 10 to 100, more preferably from 20 to 70 nucleotides. The appropriate length for primers and probes under a particular set of assay conditions may be empirically determined by one of skill in the art. The formation of stable hybrids depends on the melting temperature (Tm) of the DNA. The Tm depends on the length of the primer or probe, the ionic strength of the solution and the G+C content. The higher the G+C content of the primer or probe, the higher is the melting temperature because G:C pairs are held by three H bonds whereas A:T pairs have only two.

For amplification purposes, pairs of primers with approximately the same Tm, preferably identical Tm, are preferable. Primers may be designed using the OSP software (Hillier and Green, (1991) PCR Methods Appl., 1: 124-8, the disclosure of which is incorporated by reference in its entirety), based on GC content and melting temperatures of oligonucleotides, or using PCR are based on the octamer frequency disparity method (Griffais *et al.*, (1991) Nucleic Acids Res. 19: 3887-3891, the disclosure of which is incorporated by reference in its entirety), or any other methods or algorithm known to those skilled in the art.

As an illustration, primers and probes for carrying out a real-time PCR may be chosen using the Primer Express (Applied Biosystems) software according to the following criteria. Tm is chosen to range from 58°C to 60°C, preferably 59°C. The GC percentage is chosen to range between 40 and 60%. The primer length is chosen to range between 15 and 25 base pairs, preferably 20 base pairs. In addition, primers having more than 2 cytosine or guanine residues within the last 5 nucleotides of their 3' end are eliminated as well as couples of primers susceptible of forming intermolecular or secondary structure base-pairing as can be deduced from their primary structure. Preferably, couples of primers are chosen in order not to be able to amplify CD38 genomic DNA. Primers of SEQ ID Nos 1 and 2 were chosen according to these criteria.

Determination of primer amplification efficiency for Real-time PCR

For competitive PCR and real-time PCR, couples of primers need to be validated experimentally according to standard methods known to those skilled in the art (see for example Bieche *et al.*, (1999) supra). For Real-time PCR, the efficiency of amplification of a couple of primers can be assessed as follows.
An optimal PCR reaction may be described as:

$$C = C0*(1+E)^{Ct}$$

where C is the concentration of DNA targets when the signal threshold is reached, C0 is the concentration of DNA targets in the initial sample before amplification, E is the efficiency of amplification and Ct the number of cycles performed when the signal threshold is reached.
To measure the efficiency E for a given couple of primers, a standard curve plotting the number of cycles numbers necessary to reach a signal threshold versus the logarithm of the target concentration is drawn from experimental results, for example, using a range of dilutions for a sample containing a known amount of target DNA.
Such standard curve Ct=f(LogC) can be described as:

$$Ct = 1/(Log(1+E) * (LogC - LogC0).$$

To have an optimized PCR, the efficiency of amplification should be as close to 1 as possible to allow for doubling of the number of DNA targets per cycle. Thus, the efficiency of couples of primers is assessed by their ability to give a standard curve with a slope of 1/Log(1+E)=3.32. Alternatively, E can be computed as $E=10^{1/m}-1$ where m is the slope of the standard curve.

*2.1.3.2. Preparation of primers and probes*

Primers and probes may be prepared by any suitable method, including, for example, cloning and restriction of appropriate sequences and direct chemical synthesis as described elsewhere in the present application.
Detection probes are generally nucleic acid sequences or uncharged nucleic acid analogs such as, for example peptide nucleic acids which are disclosed in International Patent Application WO 92/20702, morpholino analogs which are described in U.S. Patents Numbered 5,185,444; 5,034,506 and 5,142,047, which disclosures are hereby incorporated by reference in their entireties. The probe may have to be rendered "non-extendable" in that additional dNTPs cannot be added to the probe, generally by modifying the 3' end of the probe such that the hydroxyl group is no longer capable of participating in elongation. Techniques to render probes non extendable are well known to those skilled in the art including techniques to functionalize the 3' end of the probe with the capture or detection label to thereby consume or otherwise block the hydroxyl group or simply by cleaving, replacing or modifying the 3' hydroxyl group (see U.S. Patent Application Serial No. 07/049,061 filed April 19, 1993, for descriptions of modifications, which can be used to render a probe non-extendable).

*2.1.3.3. Labeling of probes*

Primers and probes according to the present invention can be labeled, if desired, by incorporating any label known in the art to be detectable by spectroscopic, photochemical, biochemical, immunochemical, or chemical means. For example, useful labels include radioactive substances (including, $^{32}$P, $^{35}$S, $^{3}$H, $^{125}$I), fluorescent dyes (including, 5-bromodesoxyuridin, fluorescein, acetylaminofluorene, digoxigenin) or biotin. Preferably, polynucleotides are labeled at their 3' and 5' ends. Examples of non-radioactive labeling of nucleic acid fragments are described in the French patent No. FR-7810975, Urdea *et al*, (1988) Nuc. Acids Res. 11:4937-4957 and Sanchez-Pescador *et al* (1988), J. Clin. Microbiol. 26:1934-1938, which disclosures are hereby incorporated by reference in their entireties. In addition, the probes according to the present invention may have structural characteristics such that they allow the signal amplification, such structural characteristics being, for example, branched DNA probes as those described in Urdea *et al.* (1991), supra, Horn *et al.*, (1991) Nucleic Acids Symp. Ser. 24:201-202 or in the European patent No. EP 0 225 807, which disclosures are hereby incorporated by reference in their entireties.
The detectable probe may be single stranded or double stranded and may be made using techniques known in the art, including *in vitro* transcription, nick translation, or kinase reactions. A nucleic acid sample containing a sequence capable of hybridizing to the labeled probe is incubated with the labeled probe. If the nucleic acid in the sample is double stranded, it may be denatured prior to incubation with the probe.

*2.1.3.4. Immobilization of primers or probes*

A label can also be used to capture the primer, so as to facilitate the immobilization of either the primer or a primer extension product, such as amplified DNA, on a solid support. A capture label is attached to the primers or probes and can be a specific binding member that forms a binding pair with the solid's phase reagent's specific binding member (e.g. biotin and streptavidin). Therefore depending upon the type of label carried by a polynucleotide or a probe, it may be employed to capture or to detect the target DNA. Further, it will be understood that the polynucleotides, primers or probes provided herein, may, themselves, serve as the capture label. For example, in the case where a solid phase reagent's binding member is a nucleic acid sequence, it may be selected such that it binds a complementary portion of a primer or probe to thereby immobilize the primer or probe to the solid phase. In cases where a polynucleotide probe itself serves as the binding member, those skilled in the art will recognize that the probe will contain a sequence or "tail" that may not be complementary to the target. In the case where a polynucleotide primer itself serves as the capture label, at least a portion of the primer will be free to hybridize with a nucleic acid on a solid phase. DNA Labeling techniques are well known to the skilled technician.

Any of the primers or probes of the present invention can be conveniently immobilized on a solid support. The solid support is not critical and can be selected by one skilled in the art. Thus, latex particles, microparticles, magnetic beads, non-magnetic beads (including polystyrene beads), membranes (including nitrocellulose or nylon strips), plastic tubes, walls of microtiter wells, glass or silicon chips, sheep (or other suitable animal's) red blood cells and duracytes are all suitable examples. Suitable methods for immobilizing nucleic acids on solid phases include ionic, hydrophobic, covalent interactions and the like. A solid support, as used herein, refers to any material that is insoluble, or can be made insoluble by a subsequent reaction. The solid support can be chosen for its intrinsic ability to attract and immobilize the capture reagent. Alternatively, the solid phase can retain an additional receptor that has the ability to attract and immobilize the capture reagent. The additional receptor can include a charged substance that is oppositely charged with respect to the capture reagent itself or to a charged substance conjugated to the capture reagent. As yet another alternative, the receptor molecule can be any specific binding member which is immobilized upon (attached to) the solid support and which has the ability to immobilize the capture reagent through a specific binding reaction. The receptor molecule enables the indirect binding of the capture reagent to a solid support material before the performance of the assay or during the performance of the assay. The solid phase thus can be a plastic, derivatized plastic, magnetic or non-magnetic metal, glass or silicon surface of a test tube, microtiter well, sheet, bead, microparticle, chip, sheep (or other suitable animal's) red blood cells, duracytes® and other configurations known to those of ordinary skill in the art. The primers or probes of the invention can be attached to or immobilized on a solid support individually or in groups of distinct polynucleotides to a single solid support.

2.2. Quantification of CD38 polypeptides present in a sample

Alternatively to methods quantifying CD38 polynucleotides in a sample, methods determining the amount of CD38 polypeptides present in a given context may be used to determine CD38 expression level in a sample as described below.
Quantification of CD38 polypeptides in a test sample can be carried out using any compound that binds to a CD38 polypeptide or a fragment thereof. Such products may be antibodies, binding fragments of antibodies, compounds able to bind specifically to CD38 polypeptides or fragments thereof, including CD38 agonists and antagonists, substrates or ligands. Examples of binding compounds include the monoclonal antibody Moon-1, the ligand CD31, sodium nitroprusside, N-ethylmaleimide.
The binding compounds may be labeled or unlabeled depending on the type of assay used. Labels which may be coupled to the antibodies include those known in the art and include, but are not limited to, enzymes (e.g., horseradish peroxidase or glucose oxidase), radioisotopes, fluorogenic (e. g., fluorescein isothiocyanate (FITC), fluorescein isocyanate (FIC), 5dimethylamine-1-napthalenesulfonyl chloride (DANSC), tetramethyl- rhodamine isothiocyanate (TRITC), lissamine, and the like), chromogenic substrates, cofactors, biotin/avidin, chemiluminescent compounds, phosphorescent compounds, colloidal gold, colored particles and magnetic particles. As is appreciated by the skilled practitioner, in such cases in which the principal indicating group is an enzyme, additional reagents (e. g., substrates) are required for the production of a visible signal. Radioactive elements of various classes, such as such as $^{124}I$, $^{125}I$, $^{128}I$, $^{132}I$ and $^{51}Cr$, (gamma ray emitters); $^{32}P$, $^{3}H$, $^{35}S$ (beta emitters), and $^{11}C$, $^{14}C$, 150, or $^{13}N$ (positron emitters), may also be used as detectable labels. The labeled complex may be detected visually, with a spectrophotometer, or by another detector, depending on the labeling or indicating group.
Modification of the binding compounds allows for coupling by any known means to carrier proteins or peptides or to known water-insoluble supports or matrices, for example, polystyrene or polyvinyl chloride microtiter plates, wells, or tubes; glass tubes or glass beads; and chromatographic supports, such as paper, cellulose and cellulose derivatives, and silica. Other suitable solid supports or matrices include the following as non limiting examples: crosslinked dextran (Pharmacia, Piscataway, NJ); agarose, polystyrene beads (about 1-5 microns in diameter; e.g., Abbott Laboratories, Illinois); and crosslinked polyacrylamide; nitrocellulose- or nylon-based webs, such as sheets, strips, paddles, or sticks. Quantification of CD38 polypeptide level in a sample may be achieved using any techniques known to those skilled in the art, including but not limiting to enzyme-linked immunosorbent assays (ELISA), radioimmunoassays (RIA), immunoblotting assays (e.g. Western blot), immunofluorescent assays, immunoprecipitation assays, chemiluminescent assays, and immunohistochemical assays.

A preferred protein quantification technique is the ELISA technique (Engvall *et al.*, (1971), Immunochemistry, 8:871-4; Stites *et al.*, (1982) in Basic and Clinical Immunochemistry, 4th Edition, Chap. 22, Lange Medical Publications, Los Altos, CA.; Reen, (1994), Methods Mol. Biol. 32:461-6; which disclosures are incorporated by reference in their entireties). The ELISA assays embraced by the present invention may be, for example, of direct format (where the labeled first antibody reacts with the antigen, e.g., CD38 polypeptide), of indirect format (where a labeled second antibody reacts with the first antibody that binds to the antigen), of competitive format (such as the addition of a labeled antigen), or of sandwich format (where both labeled and unlabelled antibodies are utilized), as well as of any other format one skilled in the art could envision.

An ELISA assay initially requires preparing an antibody specific to the antigen, i.e. said CD38 polypeptide or fragment thereof, which can be monoclonal or polyclonal. In addition, when the anti-CD38 antibody is not labeled, another antibody, so-called reporter antibody, is prepared which binds to the specific antibody, or directly to the antigen itself. To the reporter antibody is attached a detectable entity such as a radioactive, fluorescent or enzymatic reagent.

In a typical ELISA, a specific anti-CD38 antibody is first incubated on a solid support, e.g. a polystyrene well that binds the antibody permanently. Incubating with an unrelated protein such as bovine serum albumin then covers any free binding site on the wells. Next, the test sample is incubated on the antibody bound to the wells; standards with known amounts of antigen may also be included in the assay in parallel to provide a quantitative measure. After the wells are washed with buffer, a second specific anti-CD38 antibody, which either is a monoclonal or polyclonal antibody different from the first antibody used to coat the well, or is a specific antibody already conjugated to a detection reagent, is incubated in the wells. During this time the second antibody attaches to any CD38 polypeptides attached to the first antibody coated on the polystyrene well. Unbound antibody is washed out with buffer. If a reporter conjugated antibody has not already been used, a third antibody linked to a reporter molecule, e.g. horseradish peroxidase, is placed in the dish which can bind to the second antibody without binding to the first antibody used to coat the plate. Unattached reporter antibody is then washed out. The quantification of the signal provided by the reporter antibody then indicates the amount of CD38 polypeptide present in the sample. Quantitative results typically are obtained by reference to a standard curve.

*How to make such CD38-binding compounds*

Methods for producing an anti-CD38 antibody

Anti-CD38 antibodies may be prepared by any suitable method known in the art. Polyclonal anti-CD38 antibodies may be prepared using an intact CD38 polypeptide or fragments thereof as the immunogen. As will be appreciated by those having skill in the art, the immunogen can be conjugated to a carrier protein, if desired, to increase immunogenicity, particularly, if a small CD38 peptide is used. Commonly used carriers that are routinely chemically coupled to peptides include but are not limited to bovine serum albumins, thyroglobulin, hemocyanin and tetanus toxoid. The coupled immunogen-carrier is then used to immunize a recipient animal (e.g., mouse, rat, sheep, goat, or rabbit) and anti-CD38 antibodies isolated from said immunized animal, preferably from the serum of said animal.

Alternatively, anti-CD38 antibodies may be monoclonal antibodies (mAb). As used herein, the term "monoclonal antibody" is not limited to antibodies produced through hybridoma technology but it rather refers to an antibody that is derived from a single clone, including eukaryotic, prokaryotic, or phage clone, and not the method by which it is produced. Monoclonal anti-CD38 antibodies may be prepared by any conventional methods, including isolation of mAb from splenic cells of an immunized animal, hybridoma, genetically engineered monoclonal antibody or antibodies fragments or antibody produced by *in vitro* immunization by certain cells, and by phase display techniques. For example, see Kohler, *et al.*, *Nature,* 256:495, 1975; *Current Protocols in Molecular Biology;* Harlow and Lane, *Antibodies: A Laboratory Manual,* Cold Spring Harbor Laboratory, New York (1988), Hammerling, *et al*, (1981) Monoclonal Antibodies and T Cell Hybridomas, Elsevier, N.Y. 563-681). (Said references incorporated by reference in their entireties).

Antigen binding fragments such as Fab, F(ab')2 and ssFv fragments may be produced, for example, from hybridoma-produced antibodies, by proteolytic cleavage, using enzymes such as papain (to produce Fab fragments) or pepsin (to produce F(ab')2 fragments) or other proteases.

Alternatively, antibodies of the present invention can be produced through the application of recombinant DNA technology or through synthetic chemistry using methods known in the art. For example, in phage display methods, functional antibody domains are displayed on the surface of a phage particle, which carries polynucleotide sequences encoding them. Phages with a desired binding property, e.g. a CD38 polypeptide binding property, are selected from a repertoire or combinatorial antibody library (e.g. human or murine) by selecting directly with antigen, typically antigen bound or captured to a solid surface or bead. Phage used in these methods are typically filamentous phage including fd and M13 with Fab, Fv or disulfide stabilized Fv antibody domains recombinantly fused to either the phage gene III or gene VIII protein. Examples of phage display methods that can be used to make the antibodies of the present invention include those disclosed in Brinkman *et al.* (1995) *J. Immunol Methods.* 182:41-50; Ames, *et al.* (1995) *J. Immunol. Meth.* 184:177-186; Kettleborough, *et al.* (1994) Eur. L Immunol. 24:952-958; Persic, *et al.* (1997) Gene. 1879-81; Burton *et al.* (1994), *Adv. Immunol.* 57:191-280; PCT/GB91/01134; WO 90/02809; WO 91/10737; WO 92/01047; WO 92/18619; WO 93/11236; WO 95/15982; WO 95/20401; and US Patents 5,698,426, 5,223,409, 5,403,484, 5,580,717, 5,427,908, 5,750,753, 5,821,047, 5,571,698, 5,427,908, 5,516,637, 5,780,225, 5,658,727 and 5,733,743 (said references incorporated by reference in their entireties).

If necessary as assessed by one skilled in the art, polyclonal or monoclonal antibodies can be further purified, for example, by binding to and elution from a matrix to which the polypeptide or a peptide to which the antibodies were raised is bound. Those of skill in the art will know of various techniques common in the immunology arts for purification and/or concentration of polyclonal antibodies, as well as monoclonal antibodies (See for example, Coligan, *et al.*, Unit 9, *Current Protocols in Immunology,* Wiley Interscience, 1994, incorporated herein by reference).

Screening for binding compounds

Alternatively, CD38 binding compounds may be found using ligand screening methods. In such screening method, a biological sample or a defined compound to be tested as a putative ligand of a CD38 protein is brought into contact with the corresponding purified CD38 protein, for example the corresponding purified recombinant CD38 protein produced by a recombinant cell host, or fragments thereof in order to form a complex between said CD38 protein or fragments thereof and the putative ligand molecule to be tested. The compound to be tested for binding may be labeled with a detectable label, such as a fluorescent, radioactive, or enzymatic tag and placed in contact with immobilized CD38 protein, or fragments thereof under conditions that permit specific binding to occur. After removal of non-specifically bound molecules, bound molecules are detected using appropriate means.

Various candidate substances or molecules can be assayed for interaction with a CD38 polypeptide. These substances or molecules include, without being limited to, natural or synthetic organic compounds or molecules of biological origin such as polypeptides.

Interactions with drugs or small molecules, such as molecules generated through combinatorial chemistry approaches may, for example, be tested using the microdialysis coupled to HPLC method described by Wang *et al.* (1997) Chromatographia, 44 : 205-208 or the affinity capillary electrophoresis method described by Bush *et al.* (1997) ), J. Chromatogr., 777 : 311-328.

In further methods, peptides, drugs, fatty acids, lipoproteins, or small molecules able to interact with said CD38 protein or fragments thereof may be identified using assays such as affinity chromatography, competition experiments or optical biosensor methods (Edwards and Leatherbarrow (1997) Analytical Biochemistry, 246, 1-6; Szabo *et al.* (1995) Curr Opin Struct Biol 5, 699-705.

When the candidate substance or molecule comprises a polypeptide, this polypeptide may be the resulting expression product of a phage clone belonging to a phage-based random peptide library (Parmley and Smith, (1988) Gene 73: 305-318; Oldenburg *et al.*, (1992) Proc. Natl. Acad. Sci. USA 89:5393-5397; Valadon *et al.*, (1996), J. Mol. Biol., 261: 11-22; Westerink, (1995) Proc. Natl. Acad. Sci USA., 92:4021-4025; Felici *et al.*, (1991) J. Mol. Biol., 222:301-310, or alternatively the polypeptide may be the resulting expression product of a cDNA library cloned in a vector suitable for performing a two-hybrid screening assay (US Patents N° US 5,667,973 and 5,283,173 ; Harper *et al.* (1993) Cell, 75 : 805-816 ; Cho *et al.* (1998) *Proc. Natl. Acad. Sci. USA*, 95(7) : 3752-3757 ; Fromont-Racine *et al.* (1997) Nature Genetics, 16(3) : 277-282, which disclosures are hereby incorporated by reference in their entireties.

3. Quantification of CD38 activity

Alternatively to methods aiming at determining CD38 expression levels through determination of the amount of CD38 polynucleotides or of CD38 polypeptides in a sample, assays for quantification of CD38 activity may be used. Assessing CD38 activity may be performed using a variety of techniques.

NAD+glycohydrolase activity may be performed by measurement of the hydrolysis of NAD+, by spectrophotometric assays and fluorometric assays (Pfister et al, (2001), Eur. J. Biochem., 268, 5601-5608) ; cADPR hydrolase activities may be performed by measurement of the hydrolysis of cADPR, by radioimmunoassay (Takasawa et al, (1998), J. Biol. Chem., 273, 2497-2500) ; ADP-ribosyl cyclase may be performed by measurement of the cyclization of the NGD+ into cGDPR, by fluorometry (Graeff et al., (1994), J.Biol.Chem., 269(48):30260-7).

IV. Methods of diagnostic, prognostic and monitoring of COPD

Methods to determine the level of CD38 expression and/or activity may also be very useful for diagnostic, prognostic, and monitoring purposes. Indeed, an elevated CD38 expression and/or activity level may have a predictive value for COPD.

Thus, other objects of the invention concern methods of diagnosis, prognosis and monitoring of COPD for an individual. In such methods, the macrophagic CD38 expression levels and/or activity are quantified in at least one biological sample recovered from said individual using any one of the methods described herein.

In diagnosis and prognosis methods, said macrophagic CD38 expression levels and/or activity in said individual is compared to the macrophagic CD38 expression levels and/or activity of a control sample. For diagnosis purposes, control samples may be samples derived from individuals not suffering from COPD, preferably healthy individuals. Alternatively, said level of macrophagic CD38 expression and/or activity in said individual is compared to standard values for individuals not suffering from COPD, preferably healthy individuals. An elevated level of CD38 expression and/or activity is then taken as a sign of the presence of COPD.

For prognosis purposes, said individual already suffers from a disorder, preferably COPD and even more preferably at an early stage. Control samples may then be samples derived from individuals suffering from COPD, preferably at a later stage. A level of CD38 expression and/or activity similar in said individual to be tested and said control samples is then taken as a sign of the possibility for said individual to move towards a more advanced stage of the disorder. As an illustrating example, the individual may be classified at stage 0 of COPD and his macrophagic CD38 expression and/or activity level compared to the macrophagic CD38 expression and/or activity level of a patient suffering from a stage I, II, or III of COPD. Alternatively, such methods and assays may be very useful to predict chronic inflammation in a patient suffering from a disorder that is not an activated-macrophage-related disorder.

In monitoring methods, the macrophagic level of CD38 expression and/or activity is measured in different samples recovered from the same individual suffering from COPD at different time points. Levels of CD38 expression and/or activity are then compared in order to evaluate the degree of severity or the evolution of COPD.

Gene quantification is also useful to assess the efficacy of a treatment against COPD. Indeed, quantifying the CD38 expression and/or activity level in macrophages in a patient suffering from COPD and submitted to a treatment may be an indication of the ability of said treatment to interfere with the disorder.

Therefore, a further object of the invention is a method of assessing the efficacy of a treatment for an individual suffering from COPD, wherein said method comprises the step of determining whether macrophagic CD38 expression levels and/or activity in a biological sample recovered from said individual submitted to said treatment are modulated using any one of the methods described herein. In such methods, the efficacy of the treatment is assessed by comparing the macrophagic CD38 expression and/or activity levels in said individual submitted to said treatment to the macrophagic CD38 expression and/or activity levels to a control sample. Said control sample may be of two types: either a (1st type )sample derived from an individual suffering from COPD and not submitted to said treatment, preferably the same individual before he was submitted to said treatment, or a 2nd type sample derived from an individual not suffering from COPD, preferably a healthy individual. The efficacy of said treatment may be then assessed by either or both of the following criteria: i) a decreased level of the macrophagic CD38 expression and/or activity in said individual submitted to said treatment when compared to a control sample of the 1st type; ii) a difference as small as possible between the macrophagic CD38 expression and/or activity in said individual submitted to said treatment and a control of the 2nd type.

V. Screening assays for agents modulating the level of gene expression and/or activity

Compounds able to modulate (preferentially inhibit) CD38 expression may be used in the prevention or treatment of COPD. Such compounds may be found using screening assays.

In such drug screening assays, the agent to be tested is contacted with macrophages under conditions permissive for the quantification of the macrophagic CD38 expression and/or activity level. The CD38 expression and/or activity level is then measured and compared to the level of CD38 expression and/or activity of control samples. Such controls typically include macrophages not contacted with the agent. A significant change in the CD38 expression and/or activity levels in the presence of the agent relative to what is detected in the absence of the agent is indicative of a modulation of gene expression by the agent. Indeed, a decrease in CD38 expression and/or activity in samples containing the agent compared to samples not containing said agent is indicative of an agent able to reduce CD38 expression. An increase in CD38 expression and/or activity in samples containing the agent compared to sample not containing said agent is indicative of an agent able to enhance gene expression. Said assay comprises the steps of

a) contacting said agent with macrophages under conditions allowing quantification of CD38 expression and/or activity ; and

b) determining whether CD38 expression levels and/or activity are modulated in said macrophages using any method described herein.

In a first embodiment, said method is a method of determining whether an agent is able to reduce or inhibit the level of gene expression and/or activity, wherein said method comprises the steps of

a) contacting said agent with macrophages under conditions allowing quantification of CD38 expression and/or activity; and
b) determining whether CD38 expression levels and/or activity is reduced in said sample compared to the macrophagic CD38 expression and/or activity level of a control sample using any method described herein.

In a preferred embodiment, said agent is a compound able to inhibit or reduce CD38 expression and/or activity. In a first more preferred embodiment, said compound is able to bind to a CD38 polynucleotide or a fragment thereof. In a further embodiment, said compound is able to bind to regulatory sequences of said CD38 polynucleotide or fragment thereof. In another further embodiment, said compound is an antisense oligonucleotide or an oligonucleotide able to form a triple helix with said CD38 polynucleotide or fragment thereof. In a second more preferred embodiment, said compound is able to bind to a CD38 polypeptide or fragment thereof. In a further preferred embodiment, said compound able to bind to said CD38 polypeptide or fragment thereof is an anti-CD38 antibody or a fragment thereof. In another further preferred embodiment, said compound able to bind to said CD38 polypeptide or fragment thereof is a CD38 antagonist or a fragment thereof. In a third more preferred embodiment, said compound is a non functional CD38 polypeptide or a fragment thereof. In a fourth more preferred embodiment, said compound is sodium nitroprusside, N-ethylmale imide, nicotinamide 2'-deoxyriboside and/or 5-methylnicotinamide 2'deoxyriboside.

Any assay well known to those skilled in the art may be used in the context of the invention. Generally, a plurality of assay mixtures is run in parallel with different agent concentrations to obtain a differential response to the various concentrations. Typically, one of these concentrations serves as a negative control, i.e. at zero concentration or below the level of detection.

Also included in the screening method of the invention are combinatorial chemistry methods for identifying chemical compounds. See, for example, Plunkett & Ellman, ("Combinatorial Chemistry and New Drugs." *Scientific American ,* April, 69, 1997). Areas of investigation for combinatorial chemistry are the development of therapeutic treatments. Of particular interest are screening assays for agents that have a low toxicity for human cells.

A variety of reagents may be included in the screening assay. These include reagents like salts, neutral proteins, e.g. albumin, detergents, etc that are used to facilitate optimal protein--protein binding and/or reduce non-specific or background interactions. Reagents that improve the efficiency of the assay, such as protease inhibitors, nuclease inhibitors or anti-microbial agents may be used. The mixture of components is added in any order that provides for the requisite binding. Incubations are performed at any suitable temperature, typically between 4 and 40° C. Incubation periods are selected for optimum activity, but may also be optimized to facilitate rapid high-throughput screening. Typically between 0.1 and 1 hour will be sufficient.

1. Description of agents:

Definition

The term "agent" describes any molecule, particularly proteins, peptides, polypeptides, aptamers, carbohydrates, and small organic molecules. Candidate agents encompass numerous chemical classes, though typically they are organic molecules, preferably small organic compounds having a molecular weight of more than 50 and less than about 2,500 daltons (Da). Candidate agents often comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including, but not limited to: peptides, polypeptides (such as antisense polypeptides), saccharides, fatty acids, steroids, purines, pyrimidines, derivatives, structural analogs or combinations thereof.

Preparation

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification or amidification to produce structural analogs.

## 2. Screening for CD38 modulators

Of particular interest for the present invention are agents that are able to modulate CD38 expression and/or level, namely CD38 modulators. As used herein, the term "CD38 modulator" refers to both CD38 inhibitors (i.e. compounds able to reduce or inhibit CD38 expression and/or activity) and CD38 activators (i.e. compounds able to trigger or enhance CD38 expression and/or activity). Such CD38 modulators may be particularly useful in the prevention and/or treatment of COPD. Therefore other objects of the invention relate to compounds acting as CD38 modulators. Candidate compounds may be isolated in standard screening assays for compounds modulating CD38 expression and/or activity as described below.

## 2.1. Compounds acting as CD38 modulators

CD38 modulators may interfere with CD38 expression and/or activity at one or several of the different steps leading from transcription of CD38 genomic DNA to activity of the CD38 polypeptide. In particular, CD38 modulators may interact with CD38 polynucleotides and/or they may interact with CD38 polypeptides. Illustrating but not limiting examples of such compounds known to interfere with CD38 expression and/or activity are glucocorticoids, estradiol, retinoids, vitamine D3, cyclic-AMP, cytokines such as IFN-γ, Il-4 and Il-7 and transcription factors including interferon-responsive factor 1(IRF-1), nuclear factor interleukin 6 (Despande et al., (2003), FASEB journal, 17, 452-454), sodium nitroprusside, N-ethylmaleimide, nicotinamide 2'-deoxyriboside, 5-methylnicotinamide 2'deoxyriboside, the monoclonal antibody Moon-1 and the ligand CD31.

## 2.1.1. Modulators of CD38 expression

Gene expression is a complex process by which a gene's coded information (genomic DNA) is converted into structures (mostly proteins) present and operating into a cell. It involves several steps including transcription, stability, splicing, export, translation, and post-translational modifications. Each of this step is susceptible to be modulated by a compound directly through binding or indirectly.
Of particular interest as CD38 modulators are compounds able to interact with the regulatory sequences of a CD38 polynucleotide or a fragment thereof. Screening assays for such compounds are detailed below.
Other interesting compounds able to act as CD38 inhibitors are CD38 oligonucleotides used in antisense or triple helix approaches as described below.

*a) Compounds interacting with regulatory sequences of a CD38 polynucleotide.*

Of particular interest are compounds able to interact with the regulatory sequences of CD38 polynucleotides, including genomic DNA, pre-mRNA or mRNA, such as for example promoter or enhancer sequences in untranscribed regions of the genomic DNA, or regulatory sequences located in untranslated regions of CD38 mRNA. Such compounds may be isolated and eventually subsequently identified as follows.
Sequences within untranscribed or untranslated regions of a CD38 polynucleotide may be identified by comparison to databases containing known regulatory sequence such as transcription start sites, transcription factor binding sites, promoter sequences, enhancer sequences, 5'UTR and 3'UTR elements (Ghosh (2000) Nucleic Acids Res;28(1): 308-10; Perier *et al.*, (2000) Nucleic Acids Res 2000 Jan 1;28(1):302-3; Kolchanov *et al.*, (2000) Nucleic Acids Res 2000 Jan 1;28(1):298-301; Wingender *et al.*, (2001) Nucleic Acids Res.,29(1):281-3; Pesole *et al.*, (2000) Nucleic Acids Res, 28(1):193-196 which disclosures are incorporated by reference in their entireties. See Baxevanis (2001) Nucleic Acids Res 29(1):1-10 and http://www. nar.oupjournals.org for up-to-dated links to databases). Alternatively, the regulatory sequences of interest may be identified through conventional reporter vectors in which a reporter gene such as beta galactosidase, chloramphenicol acetyl transferase, green fluorescent protein, or luciferase, is under the control of CD38 sequences which regulatory function on gene expression needs to be assessed. Furthermore, such reporter vector systems allows to define whether the regulatory sequences of interest increase (CD38 activator) or decrease (CD38 inhibitor) CD38 expression. Further refinement of identified CD38 regulatory sequences may be achieved using conventional mutagenesis or deletion analyses including but not limited to site-directed mutagenesis, nested 5' and/or 3' deletions, linker scanning analysis or other techniques familiar to those skilled in the art. See for example assays described in Coles *et al.,* (1998) *Hum Mol Genet* 7:791-800, WO 97/17359, US Patent No. 5,374,544; EP 582 796; US Patent No. 5,698,389; US Patent No. 5,643,746; US Patent No. 5,502,176; and US Patent 5,266,488, which disclosures are incorporated by reference in their entireties.

Following the identification of potential CD38 regulatory sequences, proteins which interact with these regulatory sequences may be identified as described below.

Gel retardation assays may be performed independently in order to screen candidate molecules that are able to interact with the regulatory sequences of a CD38 polynucleotide or a fragment thereof, such as those described in Sambrook *et al.* (2001), supra or by Dent and Latchman (1993) The DNA mobility shift assay. In: Transcription Factors: A Practical Approach (Latchman DS, ed.) ppl-26. Oxford: IRL Press, which disclosure is hereby incorporated by reference in its entirety. These techniques are based on the principle according to which a DNA or RNA fragment that is bound to a protein migrates slower than the same unbound DNA or RNA fragment. Briefly, the target nucleotide sequence is labeled. Then the labeled target nucleotide sequence is brought into contact with either a total nuclear extract from cells containing regulation factors, or with different candidate molecules to be tested. The interaction between the CD38 target regulatory sequence and the candidate molecule or the regulation factor is detected after gel or capillary electrophoresis through retardation in migration.

Nucleic acids encoding proteins which are able to interact with the regulatory sequences of a CD38 polynucleotide may be identified using a one-hybrid system, such as that described in the booklet enclosed in the Matchmaker One-Hybrid System kit from Clontech (Catalog Ref. n° K1603-1), the technical teachings of which are herein incorporated by reference. Briefly, the target CD38 nucleotide sequence is cloned upstream of a selectable reporter sequence and the resulting polynucleotide construct is integrated in the yeast genome. Preferably, multiple copies of the target sequences are inserted into the reporter plasmid in tandem. The yeast cells containing the reporter sequence in their genome are then transformed with a library comprising fusion molecules between cDNAs encoding candidate proteins for binding onto the CD38 regulatory sequences and sequences encoding the activator domain of a yeast transcription factor such as GAL4. The recombinant yeast cells are plated in a culture broth for selecting cells expressing the reporter sequence. The recombinant yeast cells thus selected contain a fusion protein that is able to bind onto the CD38 target regulatory sequence. Then, the cDNAs encoding the fusion proteins are sequenced and may be cloned into expression or transcription vectors *in vitro.* The binding of the encoded polypeptides to the target CD38 regulatory sequences may be confirmed by techniques familiar to the one skilled in the art, such as gel retardation assays or DNAse protection assays well known to those skilled in the art. The ability of such polypeptides to effectively regulate CD38 expression may also be confirmed using techniques known to those skilled in the art including co-transfection experiments with a reporter vector system bearing the CD38 regulatory sequences of interest and an expression vector for the polypeptide to be tested.

*b) Antisense and triple helix CD38 oligonucleotides*

CD38 expression may also be inhibited or reduced either *in vitro* or *in vivo* using one or several CD38 oligonucleotide fragments as an antisense tool or a triple helix tool that binds to a CD38 polynucleotide, thereby preventing further CD38 expression.

Antisense approach

In antisense approaches, nucleic acid sequences complementary to a CD38 polynucleotide hybridize to the CD38 polynucleotide intracellularly, thereby blocking the expression of the CD38 polypeptide encoded by the CD38 polynucleotide. The antisense nucleic acid molecules to be used in such approaches may be either DNA molecules, RNA molecules or hybrid DNA/RNA molecules.

Generally, antisense nucleic acid molecules are 7-25 long oligonucleotides that are complementary to a CD38 polynucleotide, preferably a CD38 mRNA, or a fragment thereof. A combination of different antisense polynucleotides complementary to different parts of the desired targeted gene can also be used. Antisense oligonucleotides may be designed to be complementary to the 5'end of CD38 mRNA, preferably to the translation initiation codon. Alternatively, they may be complementary to a sequence of CD38 genomic DNA containing a splicing donor or acceptor site. Optimal selection of antisense oligonucleotides may also be performed using the teaching of Green *et al.*, (1986) Ann. Rev. Biochem. 55:569-597; Izant and Weintraub, (1984) Cell 36(4):1007-15 ; Wagner *et al.*, (1996) Nat Biotechnol. 14(7): 840-4), Mir and Southern (1999) Nat. Biotechnol. 17:788-792; Patzel *et al.* (1999) Nuc. Acids Res. 27:4328-4334; Lebedeva and Stein (2001) Anu; Rev. Pharmacol. Toxicol. 41:403-419; Smith *et al.*, (2000) Eur. J. Pharm. Sci. 11(3): 191-198; Sohail and Southern (2000) Adv. Drug. Deliv. Rev. 44(1):23-34, which disclosures are incorporated by reference in their entireties. The antisense nucleic acids should have a length and melting temperature sufficient to permit formation of an intracellular duplex having sufficient stability to inhibit the expression of a CD38 polynucleotides in the duplex. The antisense polynucleotides of the invention may have a 3' polyadenylation signal that has been replaced with a self-cleaving ribozyme sequence, such that RNA polymerase II transcripts are produced without poly(A) at their 3' ends, these antisense polynucleotides being incapable of export from the nucleus, such as described by Liu *et al.*, (1994) Proc. Natl. Acad. Sci. USA. 91: 4528-4262, which disclosure is hereby incorporated by reference in its entirety. In a preferred embodiment, these CD38 antisense polynucleotides also comprise, within the ribozyme cassette, a histone stem-loop structure to stabilize cleaved transcripts against 3'-5' exonucleolytic degradation, such as the structure described by Eckner *et al.*, (1991) EMBO J. 10:3513-3522, which disclosure is hereby incorporated by reference in its entirety.

Antisense polynucleotides may be obtained using different techniques well known to those skilled in the art. For example, antisense polynucleotides may be obtained by reversing the orientation of the targeted CD38 region, for example the CD38 coding region in the case a mRNA is targeted, with respect to a promoter so as to transcribe the opposite strand from that which is normally transcribed in the cell. The antisense nucleic acid molecules may then be transcribed *using in vitro* transcription systems such as those which employ T7 or SP6 polymerase to generate the transcript. Alternatively, the antisense nucleic acid molecules may then be transcribed *in vivo* by operably linking DNA containing the antisense sequence to a promoter in a suitable expression vector. Alternatively, antisense oligonucleotides of a desired sequence may be chemically synthesized using any techniques known to those skilled in the art including those described herein.

In some embodiments, antisense polynucleotides are modified to increase stability, make them less sensitive to RNase activity and/or decrease their potential toxicity. Examples of modifications suitable for use in antisense strategies include modification in the phosphate backbone, the sugar moiety, and/or the nucleobase (McKay *et al.* (1996) *Nucleic Acids Res* 24: 411-7; Monia *et al.*,. (1993) *J Biol Chem* 268: 14514-22; Crooke (1997) *Ciba Found Symp* 209: 158-164.; Monia (1997) *Ciba Found Symp* 209: 107-119.; Flanagan *et al.*, (1999) *Proc Natl Acad Sci USA* 96:3513-8; Flanagan *et al.*, (1999) *Nat Biotechnol* 17: 48-52), morpholino oligonucleotides (Summerton and Weller (1997) *Antisense Nucleic Acid Drug Dev* 7: 187-95), chemically conjugated oligonucleotides (Spiller *et al.*, (1998) *Blood* 91: 4738-46), peptide nucleic acids (Good and Nielsen (1997) *Antisense Nucleic Acid Drug Dev* 7: 431-7; Nielsen (1999) *Curr Opin Struct Biol* 9: 353-7), and plasmid-derived RNA (Weiss *et al.*, (1999) *Cell Mol Life Sci* 55: 334-58; Inouye *et al.*, (1997) *Ciba Found Symp* 209: 224-233), which disclosures are incorporated by reference in their entireties. Further examples are described in Rossi *et al.*, (1991) Pharmacol. Ther. 50:245-254 ; in Herdewijn (2000) antisense Nucleic Acid Drug Dev. 10(4) :297-310 ; and in Lebedeva and Stein (2001), supra, which disclosures are incorporated by reference in their entireties.

The appropriate level of antisense nucleic acids required to inhibit gene expression may be determined *using in vitro* expression analysis. The antisense polynucleotides may be introduced into test cells or organisms by diffusion, injection, infection or transfection using procedures known in the art. For example, the antisense nucleic acids can be introduced into the body as a bare or naked polynucleotide, polynucleotide encapsulated in lipid, polynucleotide sequence encapsidated by viral protein, or as an polynucleotide operably linked to a promoter contained in an expression vector. The expression vector may be any of a variety of expression vectors known in the art, including retroviral or viral vectors, vectors capable of extrachromosomal replication, or integrating vectors. The vectors may be DNA or RNA. Such delivery systems include cationic lipids (Hope *et al.*, (1998) *Mol Membr Biol* 15: 1-14; Gokhale *et al.*, (1997) *Gene Ther* 4: 1289-99) molecular umbrellas (Janout *et al.*, (1997) *Bioconjug Chem* 8: 891-5), water-soluble cationic porphyrins (Benimetskaya *et al.*., (1998) *Nucleic Acids Res* 26: 5310-7; Talkle *et al.*, (1997) *Antisense Nucleic Acid Drug Dev* 7: 177-85), Starburst polyamidoamine dendrimers (Helin *et al.*, (1999) *Biochem Pharmacol* 58: 95-107; Bielinska *et al.*, (1996) *Nucleic Acids Res* 24: 2176-82); lipophilic conjugates (Rump *et al.*, (1998) *Bioconjug Chem* 9: 341-9; Spiller *et al.*, (1998) *Blood* 91: 4738-468), fusion peptides containing both hydrophobic and hydrophilic sequences (Chaloin *et al.*, (1998) *Biochem Biophys Res Commun* 243: 601-8). For reviews of such delivery systems see Juliano and Yoo (2000) Curr. Opin. Mol. Ther. 2(3):297-303; Garcia-Chaumont *et al.*, (2000) Pharmacol. Ther. 87(2):255-277; Lebedeva *et al.*, (2000) Eur. J. Pharm. Biopharm. 50(1):101-119, which disclosures are incorporated by reference in their entireties).

The antisense molecules are introduced onto cell samples at a number of different concentrations preferably between $1 \times 10^{-10}$M to $1 \times 10^{-4}$M. Once the minimum concentration that can adequately control gene expression is identified, the optimized dose is translated into a dosage suitable for *use in vivo.* For example, an inhibiting concentration in culture of $1 \times 10^{-7}$ translates into a dose of approximately 0.6 mg/kg bodyweight. Levels of oligonucleotide approaching 100 mg/kg bodyweight or higher may be possible after testing the toxicity of the oligonucleotide in laboratory animals. It is additionally contemplated that cells from the vertebrate are removed, treated with the antisense oligonucleotide, and reintroduced into the vertebrate. In a preferred application of this invention, the effectivenes of antisense inhibition on translation is monitored using techniques to quantify not only the amount of CD38 polynucleotides but also the amount of CD38 polypeptides and/or the amount of CD38 activity.

An alternative to the antisense technology that is used according to the present invention comprises using ribozymes that will bind to a target sequence via their complementary polynucleotide tail and that will cleave the corresponding CD38 target RNA (pre-mRNA or preferably mRNA) by hydrolyzing its target site (namely "hammerhead ribozymes").

Briefly, the simplified cycle of a hammerhead ribozyme comprises (1) sequence specific binding to the CD38 target RNA via complementary antisense sequences; (2) site-specific hydrolysis of the cleavable motif of the target strand; and (3) release of cleavage products, which gives rise to another catalytic cycle. Indeed, the use of long-chain antisense polynucleotide (at least 30 bases long) or ribozymes with long antisense arms are advantageous. A preferred delivery system for antisense ribozyme is achieved by covalently linking these antisense ribozymes to lipophilic groups or to use liposomes as a convenient vector.

Ribozyme activity can be optimized as described by Stinchcomb *et al.*, "Method and Composition for Treatment of Restenosis and Cancer Using Ribozymes," filed May 18, 1994, U.S. Ser. No. 08/245,466. The details will not be repeated here, but include altering the length of the ribozyme binding arms (stems I and III, see FIG. 2c), or chemically synthesizing ribozymes with modifications that prevent their degradation by serum ribonucleases (see e.g., WO 92/07065; Perrault *et al.*, (1990) *Nature* 344, 565; Pieken *et al.*, (1991) *Science* 253, 314; Usman and Cedergren, (1992) *Trends in Biochem. Sci.* 17, 334; WO 93/15187; and WO 91/03162, as well as U. S. Patent Application 07/829,729, and EP 92110298.4 which describe various chemical modifications that can be made to the sugar moieties of enzymatic RNA molecules. Modifications which enhance their efficacy in cells, and removal of stem II bases to shorten RNA synthesis times and reduce chemical requirements. (All these publications are hereby incorporated by reference herein.).

Antisense ribozymes according to the present invention may be prepared and used as described by Rossi *et al.*, (1991) Pharmacol. Ther. 50:245-254 ; Sczakiel *et al.*, (1995) Trends Microbiol. 3(6):213-217, Arndt and Rank (1997) *Genome* 40: 785-97; Rossi (1997) *Ciba Found Symp* 209: 195-204; Kohler *et al.*, (1999) *J Mol Biol* 285: 1935-50; Lewin and Hauswirth (2001) Trends Mol Med 7(5):221-8; Phylactou (2000) Adv Drug Deliv Rev;44(2-3):97-108; Amarzguioui and Prydz (1998) Cell Mol Life Sci; 54(11):1175-202; U.S. Pat. No. 5,254,678; US Pat. No. 6,307,041, Bertrand. *et al.*, (1994) Nucleic Acids Res. 22:293-300 , US RE37411, US Patent 6,280,936, US Patent No. 6,271,359, US Patent No. 6,265,167, US Publication No. 20010006801, US Patent No. 6,204,027, US Patent No. 6,183,959, EP 1144599 and EP 1088086 which disclosures are incorporated by reference in their entireties.

Triple Helix Approach

CD38 expression may also be inhibited using intracellular triple helix approaches. Such triple helix polynucleotides are able to inhibit transcription and are particularly useful for studying alterations in cell activity when it is associated with a particular gene. Traditionally, homopurine sequences were considered the most useful for triple helix strategies. However, homopyrimidine sequences can also inhibit gene expression. Such homopyrimidine oligonucleotides bind to the major groove at homopurine:homopyrimidine sequences. Thus, both types of sequences from the CD38 genomic DNA are contemplated within the scope of this invention.

To carry out gene therapy strategies using the triple helix approach, the CD38 genomic DNA sequence is first scanned to identify 10-mer to 20-mer homopyrimidine or homopurine stretches which could be used in triple-helix based strategies for inhibiting CD38 expression. Following identification of candidate homopyrimidine or homopurine stretches, their efficiency in inhibiting CD38 expression is assessed by introducing varying amounts of oligonucleotides containing the candidate sequences into tissue culture cells which express CD38. For information on the generation of oligonucleotides suitable for triple helix formation see Griffin *et al.*, (1989) Science 245:967-971, Casey and Glazer (2001) Prog Nucleic Acid Res Mol Biol;67:163-92 and US Patent No US6303376; which disclosures are incorporated by reference in their entireties.

The oligonucleotides can be introduced into the cells using a variety of methods known to those skilled in the art, including but not limited to calcium phosphate precipitation, DEAE-Dextran, electroporation, liposome-mediated transfection or native uptake.

Treated cells are then monitored for altered cell function or reduced CD38 expression and/or activity using techniques such as Northern blotting, RNase protection assays, or PCR based strategies to monitor the transcription levels of the CD38 gene in cells which have been treated with the oligonucleotide.

The oligonucleotides which are effective in inhibiting gene expression in tissue culture cells may then be introduced *in vivo* using the techniques and at a dosage calculated based on the *in vitro* results, as described in the section entitled "Antisense approach".

In some embodiments, the natural (beta) anomers of the oligonucleotide units can be replaced with alpha anomers to render the oligonucleotide more resistant to nucleases. Further, an intercalating agent such as ethidium bromide, or the like, can be attached to the 3' end of the alpha oligonucleotide to stabilize the triple helix.

*c) RNA interference*

CD38 expression may also be inhibited using the RNA interference (RNAi) method. RNA interference is the process of sequence-specific, posttranscriptional gene silencing in animals and plants initiated by double-stranded RNA that is homologous to the silenced gene. Molecules of small interfering RNAs (siRNAs) are 21- to 23- nucleotide RNAs, with characteristic 2- to 3- nucleotide 3'-overhanging ends resembling the RNAse III processing products of long double stranded RNAs (dsRNAs) that normally initiate RNAi. When introduced into a cell, they assemble with yet-to-be-identified proteins of an endonuclease complex (RNA-induced silencing complex), which then guides target mRNA cleavage. As a consequence of degradation of the targeted mRNA, cells with a specific phenotype characteristic of suppression of the corresponding protein product are obtained. The small size of siRNAs, compared with traditional antisense molecules, prevents activation of the dsRNA-inducible interferon system present in mammalian cells. This avoids the nonspecific phenotypes normally produced by dsRNA larger than 30 base pairs in somatic cells.

The main limitation for the moment is the transient nature of siRNA transfer into cells. Ultimately, the possibility of stable expression of siRNAs and the recent silencing gene expression using a vector-based siRNA expression system might pave the road for new gene therapy applications (Miyagishi et Taira, Nature Biotechnology (2002), 20,497-500; Lee et al, Nature Biotechnology (2002), 20, 500-505 ; Tuschl, Nature Biotechnology (2002), 20, 505-508 ; Paul et al, Nature Biotechnology (2002), 20, 446-448.

### 2.1.2. Modulators of CD38 activity

Compounds able to modulate CD38 activity may also be compounds able to interact with a CD38 polypeptide or a fragment thereof either directly (e.g. through binding) or indirectly (e.g., by modulating the ability of a CD38 polypeptide to perform its activity). Such compounds may either be CD38 inhibitors or CD38 activators.

Such a CD38 inhibitor may be an antibody, or a fragment thereof, specific for a CD38 protein or a functional portion of CD38. Illustrating but not limiting examples of anti-CD38 antibodies are IB4 (Malavasi et al., (1984), Hum. Immunol., 9, 9-12), clone T16 (Jackson and Bell, (1990), J. Immunol., 144, 2811-2815), SUN 4B7 (Deaglio et al, (1996), J. Immunol., 156, 727-734), OKT10 (Reinherz et al, (1980), Proc. Natl. Acad. Sci. USA, 77, 1588-1592). Alternatively, anti-CD38 antibodies and fragments thereof may be produced using technologies described elsewhere in the application and then tested for their ability to inhibit and/or reduce CD38 activity as described below.

Alternatively, a CD38 inhibitor may be a compound other than an antibody (e.g., small organic molecule, protein or peptide) that binds to a CD38 protein or fragment thereof and blocks its activity, i.e. an antagonist or a fragment thereof. Directions to screen for CD38 ligands are described in detail below. Such ligands may then be tested for their ability to inhibit and/or reduce CD38 activity.

Alternatively, a CD38 inhibitor may be a compound that binds to or interacts with a molecule that normally binds to or interacts with a CD38 protein, thus preventing said CD38 protein from exerting the effects it would normally exert. Illustrating but not limiting examples of CD38 inhibitors are preferably sodium nitroprusside, N-ethylmaleimide, nicotinamide 2'-deoxyriboside, 5-methylnicotinamide 2'-deoxyriboside. For example, such a CD38 inhibitor may be a compound that mimics CD38 structurally, but lacks its function such as a mutated CD38 polypeptide or a fragment thereof. Directions to synthesize such mutated non-functional CD38 polypeptides are described in detail below.

Compounds able to act as CD38 activators may be compounds (e.g., small organic molecules, proteins or peptides) that bind to a CD38 protein or fragment thereof and trigger or enhances its activity, i.e. an agonist or a fragment thereof. Illustrating but not limiting examples of CD38 agonists are all-trans retinoic acid, (IFN)-beta, and dbcAMP. Directions to screen for CD38 ligands are described in detail below. Such ligands may then be tested for their ability to trigger and/or increase CD38 activity.

Alternatively, a CD38 activator may be a compound that binds to or interacts with a molecule that normally binds to or interacts with a CD38 protein, thus triggering said CD38 protein to exerting its effects. Illustrating but not limiting example of CD38 activator is fMLP (Partida-Sanchez et al, (2001), Nature Medicine, vol. 7, 11, 1209-1216.

### a) Ligand screening methods

CD38 ligands may be screened in so-called "ligand screening assays" using techniques well known to those skilled in the art. In such ligand screening methods, a biological sample or a defined molecule to be tested as a putative ligand of a CD38 protein is brought into contact with CD38 proteins or fragments thereof under conditions to allow the formation of a complex between said CD38 protein or fragment thereof and the putative ligand molecule to be tested. Depending on the type of assays used and on whether said CD38 protein is secreted wholly (e.g. in the extracellular medium), partially (e.g. present at the membrane) or not at all (e.g. intracellular protein), different sources for said CD38 protein or fragment thereof may be used including purified recombinant CD38 protein, whole cells or cellular extracts of cells expressing CD38 proteins, or extracellular medium of CD38-expressing cells.

Various candidate ligands can be assayed for interaction with a CD38 polypeptide or fragment thereof. These substances or molecules include, without being limited to, natural or synthetic organic compounds or molecules of biological origin such as polypeptides. Compounds may be identified from a variety of sources, for example, cells, cell-free preparations, chemical libraries, and natural product mixtures. Different types of assays are available depending on the type of compounds to be tested.

As an illustrative example, to study the interaction of a CD38 protein or a fragment thereof with drugs or small molecules, such as molecules generated through combinatorial chemistry approaches, the microdialysis coupled to HPLC method described by Wang *et al.*, (1997), Chromatographia, 44 : 205-208, or the affinity capillary electrophoresis method described by Bush *et al.*, (1997), J. Chromatogr., 777 : 311-328, the disclosures of which are incorporated by reference, can be used.

In further methods, peptides, drugs, fatty acids, lipoproteins, or small molecules that interact with a CD38 protein, or a fragment thereof may be identified using assays such as the followings. The molecule to be tested for binding is labeled with a detectable label, such as a fluorescent, radioactive, or enzymatic label and placed in contact with immobilized CD38 protein or a fragment thereof under conditions allowing specific binding to occur. After removal of non-specifically bound molecules, bound molecules are detected using appropriate means.

Such candidate ligands may also be screened using competition experiments, by affinity chromatography, by optical biosensor methods or any other techniques known to those skilled in the art. When the candidate ligand comprises a polypeptide, this polypeptide may be the resulting expression product of a phage clone belonging to a phage-based random peptide library, or alternatively the polypeptide may be the resulting expression product of a cDNA library cloned in a vector suitable for performing a two-hybrid screening assay.

A. Candidate ligands screened from random peptide libraries

In such screening methods, the putative ligand is the expression product of a DNA insert contained in a phage vector. Specifically, random peptide phages libraries encoding peptides of 8 to 20 amino acids in length are generally used.

The recombinant phage population is brought into contact with an immobilized CD38 protein or fragment thereof. Then the preparation of complexes is washed in order to remove non-specifically bound recombinant phages. The phages that bind specifically to the CD38 protein are then eluted using a buffer with an acid pH or immunoprecipitated by an anti-CD38 antibody. The recovered phage population is subsequently amplified by an over-infection of bacteria (for example E. coli). The selection step may be repeated several times, preferably 2-4 times, in order to select for more specific recombinant phage clones.

Finally, the peptide produced by each recombinant phage clone is characterized by expression in infected bacteria and isolation, by expression of the phage insert in another host-vector system, or by sequencing the phage insert.

Teachings to build random peptide libraries and use them in screening assays may be found in Parmley and Smith, (1988) Gene 73: 305-318; Cortese *et al* (199) Curr Opin Biotechnol; 7(6):616-21; Oldenburg *et al.*, (1992), Proc. Natl. Acad. Sci. USA 89:5393-539; Valadon *et al.*, (1996), J. Mol. Biol., 261:11-22; Westerink, (1995), Proc. Natl. Acad. Sci USA., 92:4021-4025; Felici, (1991), J. Mol. Biol., 222:301-310; US Patent Nos 6,180,343 , US 5,834,318 ; US 5,747,334 ; US 5,564,325, US 6,107,059, US 5,733,731, US 5,723,286 ; US 5,498,530 EP 0990051, WO 9846 796, which disclosures are hereby incorporated by reference in their entireties.

B. Candidate ligands screened using competition experiments.

Compounds such as peptides, drugs or small molecules that bind to a CD38 protein or fragment thereof may be identified in competition experiments. In such assays, the CD38 protein, or a fragment thereof, is immobilized to a surface, such as a plastic plate. Increasing amounts of the compounds to be tested are placed in contact with the immobilized CD38 protein, or a fragment thereof, in the presence of a detectable labeled known CD38 protein ligand. For example, the CD38 ligand may be detectably labeled with a fluorescent, radioactive, or enzymatic tag. The ability of the test molecule to bind the CD38 protein, or a fragment thereof, is determined by measuring the amount of detectably labeled known ligand bound in the presence of the test molecule. A decrease in the amount of known ligand bound to the CD38 protein, or a fragment thereof, when the test molecule is present indicated that the test molecule is able to bind to the CD38 protein, or a fragment thereof. Teachings to practise such competitive assays may be found in US 5,434,052, WO 0171314, WO 0079260, US 6,322,992, and in US 2001 0026944, which disclosures are incorporated by reference in their entireties

C. Candidate ligands screened using affinity chromatography.

Proteins or other molecules interacting with a CD38 protein, or fragment thereof, may also be screened using affinity columns onto which the CD38 protein or fragment thereof is bound using conventional techniques, e.g. chemical coupling to a suitable column matrix such as agarose, Affi Gel®, or other matrices familiar to those of skill in art. In such methods, the affinity column may contain chimeric proteins comprising the CD38 protein or fragment thereof fused to glutathion S transferase (GST). A mixture of compounds, such as a cellular protein extract, is then run through the affinity column. Proteins or other molecules interacting with the immobilized CD38 protein or fragment thereof attached to the column can then be isolated and analyzed, for example using 2-D electrophoresis gel as described in Ramunsen *et al.*, (1997), Electrophoresis, 18: 588-598, the disclosure of which is incorporated by reference. Alternatively, the proteins retained on the affinity column can be purified using electrophoresis-based methods and then sequenced (see for example Evans *et al.*, (1996) Nat Biotechnol 14(4):504-7; Huang and Carbonell, (1999) Biotechnol. Bioeng. 20;63(6):633-41, which disclosures are incorporated by reference in their entireties), The same method can be used to isolate antibodies, to screen phage display products, or to screen phage display human antibodies.

D. Candidate ligands obtained by optical biosensor methods

Proteins interacting with a CD38 protein or fragment thereof can also be screened using an Optical Biosensor as described in Edwards and Leatherbarrow, (1997) Analytical Biochemistry, 246, 1-6 and also in Szabo *et al.*, (1995) Curr Opin Struct Biol 5, 699-705, the disclosures of which are incorporated by reference. This technique allows the detection of interactions between molecules in real time, without the need of labeled molecules.
For screening of candidate ligand molecules, the CD38 protein, or a fragment thereof, is immobilized onto a surface (such as a carboxymethyl dextran matrix). Then, a solution containing either a compound to be tested or a mixture of compounds such as a cellular extract flows onto the surface onto which the CD38 protein or fragment thereof is bound. Binding of a compound to the CD38 protein or fragment thereof, is detected as a change of the surface plasmon resonance signal. The candidate molecules tested may be proteins, peptides, carbohydrates, lipids, or small molecules generated by combinatorial chemistry. This technique may also be performed with immobilized eukaryotic or prokaryotic cells or lipid vesicles exhibiting an endogenous or a recombinantly expressed CD38 protein at their surface.
The main advantage of the method is to allow the determination of the association rate between the CD38 protein or fragment thereof and interacting compounds. It is thus possible to select specifically ligand molecules interacting with the CD38 protein, or a fragment thereof, with desired association and dissociation kinetics.

E. Candidate ligands screened through a two-hybrid screening assay.

Proteins able to bind to CD38 proteins or fragments thereof may also be screened using two-hybrid assays and its derivatives such as those described in Fields and Song (1989) Nature 340:245; Bram *et al.*, (1993), *Mol. Cell Biol.*, 13 : 4760-4769; Harper *et al.*, (1993), Cell, 75 : 805-816 ; Fields and Sternglanz (1994) Trends in Genetics 10: 286-292; Cho *et al.*, (1998), *Proc. Natl. Acad. Sci. USA,* 95(7) : 3752-3757; Fromont-Racine *et al.*, (1997), Nature Genetics, 16(3) : 277-282 ; Bartel *et al.* (1993) Biotechniques 14:920-924, Karimora *et al.*, (1998) Proc. Nad. Acad. Sci. USA 95(10):5752-5756; WO 9410300, WO 99/28746, WO0173108, US 20010024794, US 5,667,973, US 5,283,173, US 6,251,676,US 6,2051,381, which disclosures are incorporated by reference in their entireties. Alternatively, kits such as the Matchmaker Two Hybrid System 2 (Catalog No. K1604-1, Clontech) may be used as described in the manual accompanying the kit, the disclosure of which is incorporated herein by reference.
That system utilizes chimeric genes and detects protein-protein interactions via the activation of reporter-gene expression. Reporter gene expression occurs as a result of reconstitution of a functional transcription factor caused by the association of fusion proteins encoded by the chimeric genes. Indeed, two-hybrid assays rely upon the fact that the DNA binding and polymerase activation domains of many transcription factors, such as GAL4 , can be separated and then rejoined to restore functionality.
Typically, polynucleotides encoding two hybrid proteins are constructed and introduced into a yeast host cell. The first hybrid protein consists of the yeast Gal4 DNA-binding domain fused to a CD38 protein or fragment thereof (often referred to as the "bait"). The second hybrid protein consists of the Gal4 activation domain fused to a polypeptide sequence of a second protein to be tested (often referred to as the "prey"). Polynucleotides encoding proteins to be tested may be issued from DNA libraries, preferably cDNA libraries. Binding between the two hybrid proteins reconstitutes the Gal4 DNA-binding domain with the Gal4 activation domain, which leads to the transcriptional activation of a reporter gene (e.g., lacZ, URA3 or HIS3 ), which is operably linked to a Gal4 binding site.

*b) Non functional CD38 polypeptides:*

Non functional CD38 polypeptides are polypeptides that are very similar to CD38 polypeptides as described herein in the Definition section but that do not exhibit a substantial CD38 activity, namely polypeptides that present a CD38 biological activity.
Such non functional CD38 polypeptides may arise naturally or may be obtained using mutagenesis techniques well known to those skilled in the art. Recombinant DNA technology can be used to create novel mutant proteins or muteins including single or multiple amino acid substitutions, deletions, additions, or fusion proteins.

Particularly interesting non functional CD38 polypeptides able to inhibit normal CD38 activity are those fragments of CD38 polypeptides that retain the ability to interact with CD38 ligands but that are deprived of functional domains for exerting CD38 activity.

Standard techniques to mutate proteins include N-terminal and/or C-terminal deletions. Generally, such deletions are progressive in order to determine the minimal region that should be deleted in order to diminish significantly, or abolish, a desired CD38 activity.

Other mutations in addition to N- and C-terminal deletion forms of the protein discussed above may be carried out in order to decrease or abolish a desired CD38 activity. Such mutations are generally those that affect the structure, or the function of a CD38 polypeptide or both. If such mutations are contemplated, it should be remembered that there are critical areas on the protein that determine activity. In order to determine such critical residues, an approach for studying the tolerance of an amino acid sequence to changes (See, Bowie *et al.* (1994) supra) that relies on the process of evolution may be used.

Domains and/or critical amino acids in the CD38 proteins of the present invention that are essential for function can also be identified by methods known in the art such as site-directed mutagenesis (Braisted and Wells, (1996) Proc. Natl. Acad. Sci. USA, 93:5688-5692; Wells (1996) Proc. Natl. Acad. Sci. USA, 93:1-6), alanine-scanning mutagenesis (Cunningham *et al.* (1989), Science 244:1081-1085, Clackson and Wells (1995) Science, 267(5196):383-6; Leung and Hall (2000) Trends Cardiovasc. Med. 10(2):89-92), cysteine-scanning mutagenesis (Friligos *et al.* (1998) Faseb J. 12: 1281-1299), and pentapeptide scanning mutagenesis (Hayes and Hallet (2000) Trends Microbiol; 8(12):571-7), which disclosures are incorporated by reference in their entireties). In such techniques, amino acid changes genetic engineering are introduced at specific positions of a cloned gene and the resulting mutant molecules are then tested for biological activity using assays appropriate for measuring the function of the particular protein in order to identify sequences that maintain functionality. The studies indicate which amino acid changes at certain position sof the CD38 protein are likely to affect CD38 activity.

Alternatively, libraries of nucleic acids encoding mutated CD38 polypeptides generated by random mutagenesis may be employed to reveal principles of protein structure. For example, cassette mutagenesis has been used to probe the "information content" of polypeptide sequences (Reidhaar-Olson and Sauer (1988) Science, 241(4861):53-7; Davidson and Sauer (1994) Proc Natl Acad Sci, 91(6):2146-50; Davidson *et al.,* (1995) Nat Struct Biol, 2 (10) p856-64, which disclosures are incorporated by reference in their entireties). These studies involve the construction of polypeptide mutants composed of random combinations of selected amino acids. The mutants are then analyzed for their thermal denaturation properties. Current methods for creating mutant proteins in a library format include but are limited to error-prone PCR-based techniques (See e.g. Leung *et al.*, (1989) Technique 1:11-15; Caldwell and Joyce (1992) PCR Methods and Applications 2:28-33; Gramm *et al.*, (1992) Proc. Natl. Acad. Sci. USA 89:3576- 3580, which disclosures are incorporated by reference in their entireties) and cassette mutagenesis (Stemmer *et al.*, (1992) Biotechniques 14:256-265, Arkin and Youvan (1992) Proc. Natl. Acad. Sci. USA 89:7811-7815; Oliphant *et al.*, (1986) Gene 44:177-183; Hermes *et al.*, (1990) Proc. Natl. Acad. Sci. USA 87:696-700; Delagrave *et al.* (1993) Protein Engineering 6:327-331; Delgrave *et al.* (1993) Bio/Technology 11:1548-1552; Goldman, E. R. and Youvan D. C. (1992) Bio/Technology 10:1557- 1561 ), in which the specific region of a polypeptide to be tested is replaced with a synthetically mutagenized oligonucleotide. Alternatively, mutator strains of host cells may be employed to add mutational frequency (see e.g. Greener and Callahan (1995) *Strategies in Mol. Biol.* 7: 32). Preferably non-fonctional CD38 polypeptides are polypeptides in which the domains encoding the ADP ribosyl cyclase and/or ADPRc hydrolase activities are either deleted or mutated. Illustrating but not limiting examples of such non-functional CD38 polypeptides have been described in Tohgo *et al.*, (1994) *J. Biol. Chem.* 269(46):28555-7) : Mutation of C119 to K, or C201 to E induces a loss of cADPR hydrolase activity ; Mutation of C 119 to R,E,A, or C160 to A, or C 173 to A, or C201 to D,K,A induce a loss of cADPR hydrolase and ADP-ribosyl cyclase activity.

2.2. Methods of screening for compounds modulating CD38 expression and/or activity

Compounds suspected of modulating CD38 expression and/or activity such as compounds identified by the above described methods, namely compounds able to bind to regulatory sequences of CD38 polypeptides, antisense and triple helix polynucleotides, anti-CD38 antibodies, CD38 ligands and non functional CD38 polypeptides, may then be tested for their ability to effectively modulate CD38 expression and/or activity. Alternatively, libraries of compounds that have not been pre-selected using any of the methods described above, may be screened directly for their ability to modulate CD38 expression and/or activity.

Therefore, the invention also relates to methods of determining whether a compound modulates CD38 expression and/or activity.

In such screening assays, the compound to be tested is contacted with macrophages under conditions permissive for the quantification of macrophagic CD38 expression and/or activity. The CD38 expression and/or activity level is then measured and compared to the level of CD38 expression and/or activity of control samples. Such controls typically include macrophages not contacted with the compound. CD38 expression and/or activity in samples containing said compound to be tested compared to samples not containing said compound is indicative of a compound able to modulate CD38 expression and/or activity.

Examples 1-3 describe *in vitro* and *in vivo* protocols that could be used, alone or in combination, to test the ability of a compound to modulate CD38 expression and/or activity.

Screening assays for compounds modulating CD38 expression and/or activity

Screening assays for compounds modulating CD38 expression and/or activity may be of any type one skilled in the art could envision and include organisms, cells, cell lysates or cell-free systems.

*In vitro methods*

Cells expressing CD38 are incubated *in vitro* in the presence and absence of the test compound. By determining the level of CD38 expression and/or activity in the presence of the test compound and comparing it to the CD38 expression and/or activity level in the absence of test compound, compounds can be identified that modulate CD38 expression or activity. Alternatively, when the ability of a compound to interact with regulatory sequences of a CD38 polynucleotide needs to be assessed, constructs comprising a CD38 regulatory sequence operably linked to a reporter gene (e.g. luciferase, chloramphenicol acetyl transferase, LacZ, green fluorescent protein, etc.) can be introduced into host cells and the effect of the test compounds on expression of the reporter gene detected. Cells suitable for use in the foregoing assays include, but are not limited to, cells having the same origin as tissues or cell lines in which the CD38 polypeptide is known to be expressed such as macrophages, thymocyte, T lymphocytes, monocytes, NK cells, red blood cells, platelets, β-pancreatic cells and neurons or such as host cells engineered to express CD38.

*In vivo methods*

Compounds that modulate CD38 expression and/or activity can also be identified using *in vivo* screens. In these assays, the test compound is administered (e.g. IV, IP, IM, orally, or otherwise), to the animal, for example, at a variety of dose levels. The effect of the compound on CD38 expression and/or activity is determined by comparing CD38 expression and/or activity levels, for example in cells or tissues known to express the gene of interest such as macrophages. CD38 is distributed in different tissues :

pancreas (Takasawa et al, (1993), Science, 259, 370-373), heart, thyroid gland, brain (Mizuguchi et al, (1995), Brain Research, 697, 235-240) (Yamada et al, (1997), Brain Res. 756, 52-60), eyes (Khoo et Chang, (1999), Brain Res. 821, 17-25) and other human tissues (Meszaros et al, (1995), Biochem. Biophys. Res. Commun. 210, 452-456).

The effect of a compound on CD38 expression and/or activity is determined by using any of the quantifying assays described herein. Suitable test animals include rodents (e.g., mice and rats), and primates. For example, the ability of a test compound to modulate gene expression could be tested in smoking mice or LPS-stimulated mice adapting the protocols described in Examples 2 and 3. Humanized mice can also be used as test animals, that is mice in which the endogenous mouse protein is ablated (knocked out) and the homologous human protein added back by standard transgenic approaches (see techniques described herein in the "gene therapy" section). Such mice express only the human form of a protein. Humanized mice expressing only the human CD38 can be used to study *in vivo* responses of COPD in response to potential compounds regulating CD38 expression and/or activity levels.

VI. Kits

Another object of the invention is to provide kits containing necessary reagents for conducting methods according to the invention, namely, methods of determining whether an individual suffers from COPD, methods of determining whether an individual is susceptible to develop COPD, methods of monitoring an individual suffering from COPD, methods of assessing the efficacy of a treatment for an individual suffering from COPD, and methods of determining whether an agent modulates gene expression.

Such kits contain necessary reagents for carrying out any type of assays disclosed herein (hybridization-based assays, amplification-based assays, protein quantification assays, CD38 activity assays) in order to quantify CD38 expression and/or activity level in a test sample. Eventually, control samples and/or reference amounts of CD38 expression and/or activity in control conditions may be provided in order to determine whether the CD38 expression and/or activity level is elevated in said test sample compared to another situation.

Instructions for use of the kit reagents are also typically included. Instructions for use generally include a description of the reagent concentration, or at least one assay method parameter, such as the relative amounts of reagent and sample to be admixed, maintenance time periods for reagent/sample admixtures, temperature, buffer conditions, and the like.

VII. Treating and preventing COPD

The present invention further relates to methods of preventing or treating COPD by modulating, preferably decreasing CD38 activity and/or expression. Such methods use compounds acting as CD38 modulators, preferably CD38 inhibitors, such as those selected using screening protocols described herein, in pharmaceutical compositions and/or gene therapy approaches as described in the art and herein.

Pharmaceutical and physiologically acceptable compositions

The present invention also relates to pharmaceutical or physiologically acceptable compositions comprising, as active agent, the polypeptides, nucleic acids or antibodies of the invention. More particularly, the invention relates to compositions comprising, as active agent, agents and compounds selected using the above-described screening protocols such as non-functional CD38 polypeptides, anti-CD38 antibodies, CD38 antagonists or inhibitors. The invention relates to compositions comprising, as active agent, preferably CD38 inhibitors, more preferably sodium nitroprusside, N-ethylmaleimide, nicotinamide 2'-deoxyriboside, 5-methylnicotinamide 2'-deoxyriboside. Such compositions include the active agent in combination with a pharmaceutical or physiologically acceptable carrier. In the case of naked DNA, the "carrier" may be gold particles. The amount of active agent in the composition can vary with the agent, the patient and the effect sought. Likewise, the dosing regimen can vary depending on the composition and the disease/disorder to be treated.

Therefore, the invention relates to methods for the production of pharmaceutical composition comprising a method for selecting an active agent, such as a CD38 modulator, preferably a CD38 inhibitor, using any of the screening method described herein and furthermore mixing the identified active agent, with a pharmaceutically acceptable carrier.

The pharmaceutical compositions utilized in this invention may be administered by any number of routes including, but not limited to, oral, intravenous, intramuscular, intraarterial, intramedullary, intrathecal, intraventricular, transdermal, subcutaneous, intraperitoneal, intranasal, enteral, topical, sublingual, or rectal means. In addition to the active ingredients, these pharmaceutical compositions may contain suitable pharmaceutically acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co. Easton, Pa).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through a combination of active agents with solid excipient, suiting mixture is optionally grinding, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titaniumdioxide, lacquer solutions, and suitable organic solvents or solvent mixtures. Dyestuffs or pigments may be added to the tablets or dragee coatings for product identification or to characterize the quantity of active agent, i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active agents may be dissolved or suspended in suitable liquids, such as fatty oils, liquid, or liquidpolyethylene glycol with or without stabilizers.

Pharmaceutical formulations suitable for parenteral administration may be formulated in aqueous solutions, preferably in physiologically compatible buffers such as Hanks solution, Ringer's solution, or physiologically buffered saline. Aqueous injection suspensions may contain substances which increase the viscosity of the suspension, such as sodium carboxymethylcellulose, sorbitol, or dextran. Additionally, suspensions of the active agents may be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension may also contain suitable stabilizers or agents which increase the solubility of the compounds to allow for the preparation of highly concentrated solutions.

For topical or nasal administration, penetrants appropriate to the particular barrier to be permeated are used in the formulation. Such penetrants are generally known in the art.

The pharmaceutical compositions of the present invention may be manufactured in a manner that is known in the art, e.g., by means of conventional mixing, dissolving, granulating, dragee-making, levigating, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation may be a lyophilized powder which may contain any or all of the following: 1-50 mM histidine, 0.1%-2% sucrose, and 2-7% mannitol, at a pH range of 4.5 to 5.5, that is combined with buffer prior to use.

After pharmaceutical compositions have been prepared, they can be placed in an appropriate container and labeled for treatment of an indicated condition. For administration, such labeling would include amount, frequency, and method of administration.

Pharmaceutical compositions suitable for use in the invention include compositions wherein the active agent is contained in an effective amount to achieve the intended purpose. The determination of an effective dose is well within the capability of those skilled in the art.

For any compound, the therapeutically effective dose can be estimated initially either in cell culture assays, e.g., of neoplastic cells, or in animal models, usually mice, rabbits, dogs, or pigs. The animal model may also be used to determine the appropriate concentration range and route of administration. Such information can then be used to determine useful doses and routes for administration in humans.

A therapeutically effective dose refers to that amount of active agent which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Pharmaceutical compositions which exhibit large therapeutic indices are preferred.

The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active moiety or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions maybe administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

Gene therapy

Gene therapy can be used to trigger or to enhance the expression of a gene of interest, such as for example a gene encoding a CD38 inhibitor or a CD38 polypeptide, in an individual suffering from COPD. In such approaches, the coding sequence of the gene of interest can be cloned into an appropriate expression vector and targeted to a particular cell type(s), such as macrophages, to achieve an efficient high level expression using methods known to those skilled in the art. Introduction of the coding sequence for the gene of interest into target cells can be achieved, for example, using particle mediated DNA delivery, (Haynes *et al.*, (1996) J Biotechnol. 44(1-3):37-42 ; Maurer *et al.*, (1999) Mol Membr Biol. 16(1):129-40), direct injection of naked DNA, (Levy *et al.*, (1996) Gene Ther. 3(3):201-11; Felgner (1996) Hum Gene Ther. 7(15):1791-3), or viral vector mediated transport (Smith *et al.*, (1996) Antiviral Res. 32(2):99-115, Stone *et al.*, (2000) J Endocrinol. 164(2):103-18; Wu and Ataai, (2000) Curr Opin Biotechnol. 11(2): 205-8), each of which disclosures are hereby incorporated by reference in their entireties.

Alternatively, the naturally occuring regulatory sequences of the gene of interest, such as the promoter and enhancer sequences, may be replaced by stronger regulatory sequences promoting a higher expression level of said gene of interest using homologous recombination techniques well known to those skilled in the art, such as those described in Koller and Smithies(1992) *Ann. Rev. Immun.,* 10:705-730; Morrison, and Takeda (2000) Int. J. Biochem. Sci. 32:817-831; Haber (2000) Trends Genet. 16:259-264; Karran (2000) curr. Opin. Genet. Dev. 10:144-150, Crichlow and Jackson (1998) Trends Biochem. Sci. 23:394-398, Paques and Haber, (1999) Microbiol. Mol. Biol. Rev. 63:349-404, Danska and Guidos (1997) Semin. Immunol. 9:199-206, Cheah and Berhinger (2000) Methods. Mol. Biol. 136:455-463, Lanzov (1999) Mol. Genet. Metab. 68:276-282, Muller (1999) Mech. Dev. 82:3-21, Sargent and Wilson (1998) curr. Res. Mol. Ther. 1:584-692, Jasin (1996) Trends Genet. 12:224-228, Waldamn (1992) Crit. Rev. Oncol. Hematol. 12: 49-64, U.S. Patent Nos. US 5,272,071, US 6,255,113, US 6,200,812, US 6 187 305, WO 91/06666, WO 91/06667, WO 90/11354, WO 91/09955, WO 96/41 008, EP 1152058, Chan *et al.* (1999), J. Biol. Chem., 274( 17):11541-11548, Culver *et al.*, (1999), Nature Biotechnology, 17, 989-993 and using the teachings of Vasquez *et al.*, (2001) Proc. Natl. Acad. Sci. USA 98(15):8403-8410 and Lai and Lien (1999) Exp. Nephrol. 7(1):11-14, which disclosures are incorporated by reference in their entireties.

Alternatively, gene therapy may be used to decrease or inhibit the expression of an undesirable gene, such as CD38, in an individual suffering from COPD. For example, the expression of the undesirable gene may be reduced or inhibited using homologous recombination to knock-out gene expression by deleting the whole undesirable gene or part thereof such as coding sequences or regulatory sequences, or by replacing them by non-functional sequences using homologous recombination techniques known to those skilled in the art (see references describes above as well as US Patent No 6015676 and Galli-Taliadoros *et al.*, (1995) J Immunol. Methods ;181(1):1-15, which disclosures are incorporated by reference in their entireties).

Alternatively, decrease or inhibition of the expression of the undesirable gene may be achieved using an antisense or triple helix approaches as described elsewhere in the present application. For example, antisense oligonucleotides complementary to undesirable polynucleotides can be used to selectively diminish or ablate undesirable gene expression, for example, at sites of inflammation. More specifically, antisense constructs or antisense oligonucleotides can be used to inhibit the production of the undesirable gene in cells expressing high levels of undesirable proteins. Antisense mRNA can be produced by transfecting into target cells an expression vector with the undesirable gene sequence, or portion thereof, oriented in an antisense direction relative to the direction of transcription. Appropriate vectors include viral vectors, including retroviral, adenoviral, and adeno-associated viral vectors, as well as nonviral vectors. Alternatively, antisense oligonucleotides can be introduced directly into target cells to achieve the same goal. (See also other delivery methodologies described herein in connection with gene therapy.).

Tissue specific effects in gene therapy can be achieved using delivery means that are tissue-specific (e.g. viral vectors that are tissue specific in the case of virus mediated transport) or using promoter sequences that promote expression specifically in the tissue or cell of interest. For example, macrophage specific promoters may be used such as those described in Sidiropoulos *et al.*, (1997) Hum Gene Ther May 1;8(7):803-15; and Fabunmi *et al.*, (2000) Atherosclerosis Feb;148(2):375-86. Combinatorial approaches can also be used to ensure that the desired coding sequence is properly activated in the target tissue (Miller and Whelan, (1997) Hum Gene Ther. 8(7):803-15), hereby incorporated by reference in its entirety.

The therapeutic methodologies described herein are applicable to both human and non-human mammals (including cats and dogs).

VIII. Examples

Example 1 : Detection of an increased level of CD38 expression in a macrophagic cell line upon activation

Cells and culture

U937 cells (ECACCn°85011440) (these cells being referred to as non differentiated cells) are monocyte-like cells that differentiate into macrophage-like cells upon addition of a differentiating agent. U937 cells were cultured at 37°C, 5% $CO_2$, in suspension, RPMI-1640 + glutamax, 10% bovine fetal serum (Life Technologies) at pH 7.0 and cells were maintained at $0.25 \times 10^6$ to $10^6$ cells/ml. Differentiation of U937 cells into macrophage-like cells (these cells being referred to as differentiated cells) was carried out using 1 μM dibutyryl cyclic AMP (dbcAMP) for 48 hours. Differentiation was controlled by FACS analysis checking for an increased expression of CD32 and decreased expression of CD64 as previously described (Floto *et al.*, (1996) J. Physiol. 492:331-338) using anti-CD32 and anti-CD64 antibodies labeled with FITC. In addition, subsequent release of $Ca^{2+}$ from intracellular compartments in differentiated cells followed by calcium influx was then checked using fura-2 charged cells as described in Davis *et al.* (1995) Cell Calcium;17(5): 345-53. Briefly, intracellular calcium concentration was measured out using a Shimadzu Fluorometer, after charging cells by fura-2 in presence of BSA during 45 minutes at 37°C RPMI-1640. Both non differentiated U937 cells and differentiated U937 cells were washed and calcium signaling activated by adding 600 nM of thapsigargin (in RPMI 1640 pH7 in absence of serum and phenol red), an inhibitor of the endoplasmic reticulum $Ca^{2+}$-ATPase of SERCA type (Thastrup *et al.*, (1990) Proc. Natl. Acad. Sci. USA 87:2466-270). Then, the intracellular calcium level was monitored for one hour.

<u>- RNA extraction and cDNA synthesis</u>

Total RNA was extracted with Trizol (Life Technologies) from $10^7$ cells/prep. Precipitated total RNA (average yield 100μg) was taken back in 40 μl and analyzed on agarose (1%) gel stained by Ethidium bromide. cDNA synthesis was carried out using Power Script Reverse Transcriptase (Clontech) with 5μg of total RNA / in 20 μl preparation mix, corresponding to the suppliers protocol, using random hexamer primers.

<u>- Quantitative real time PCR</u>

Real time PCR was performed on a Gene Amp 5700 (Applied Biosystems) using SybrGreen technology (SybrGreen PCR Master Mix from Applied Biosystems, Part Number : 4309155). Primers of SEQ ID Nos 1 and 2 were used to amplify a portion of CD38 polynucleotides and primers of SEQ ID Nos 3 and 4 were used to amplify 18S ribosomal RNA chosen as the reference gene in those assays. Primers were used in a final concentration of 300nM with the quantity of cDNA of 2% of the cDNA preparation (corresponding to 100ng total RNA). After a preliminary step of 2 minutes at 50°C and a step of DNA polymerase activation of 10 minutes at 95°C, subsequent thermal cycles were carried out as follows: 10 seconds at 95°C to allow denaturation of double stranded cDNA and 1 minute at 60°C to allow annealing of primers and elongation of cDNA strands.

<u>- Results</u>

Results are expressed as follows. The experiment was done in the six following different cell-culture experimental conditions:

1) non differentiated non activated U937 cells, condition herein referred to as nd0h;
2) non differentiated U397 cells after 1 hour of activation, condition herein referred to as nd1h;
3) non differentiated U397 cells after 5 hours of activation, condition herein referred to as nd5h;
4) differentiated non activated U937 cells, condition herein referred to as dbc0h;
5) differentiated U937 cells after 1 hour after activation, condition herein referred to as dbc1h;
6) differentiated U937 cells after 5 hours after of activation, condition herein referred to as dbc5h.

The number of cycles upon which the signal threshold was reached for CD38 in a given experimental condition, herein referred to as Ct, was normalized by subtraction of the variation of Ct for the gene of reference, here 18S RNA, from the same RNA preparation. The geometric mean of all six experimental conditions, herein referred to as μCt, was then calculated and the relative gene expression level expressed as $2^{(\mu Ct-Ct)}$ for each experimental condition (see Table I). Relative gene expression levels of two different experimental conditions, namely x and y, are then considered to be significantly different when the ratio of relative gene expression levels, i.e. x/y, is higher than 1.7, where x has a relative gene expression level higher than y.
CD38 was thus classified as being specific for macrophage differentiation because the following criteria were met (see Table I)

a) the highest relative gene expression level of all six different experimental conditions (N) was observed in differentiated cells.
b) the highest relative gene expression level (N) was different from the highest relative gene expression level of non-differentiated cells, i.e. N/max(nd0h, nd1h, nd5h) > 1.7.

As could be seen from Table I which present results from triplicate points for 2 independent cell cultures, the highest relative gene expression level (N) was actually observed in differentiated cells. In addition, this highest relative gene expression level was different by 3.5-fold from the highest relative gene expression level of non-differentiated cells.

Table I.

| Relative CD38 expression in non-differentiated and differentiated U937 cells upon activation as assessed using real-time RT-PCR. | | | | | | | |
|---|---|---|---|---|---|---|---|
| | nd0h | nd1h | nd5h | db0h | db1h | db5h | Mean (µCt) |
| Ct | 27.99 | 27.59 | 27.48 | 25.76 | 26.19 | 26.09 | 26.85 |
| $2^{(\mu Ct-Ct)}$ | 0.5 | 0.6 | 0.6 | 2.1 | 1.6 | 1.7 | |
| N/max(nd0h,nd1h, nd5h) | 2.1/0.6 = 3.5 | | | | | | |
| Results : CD 38 expression was shown to increase during macrophage differentiation. | | | | | | | |

Example 2 : Detection of CD38 polynucleotide expression levels in an animal model for COPD

Smoking mice model

Male A/J mice aged from 6 weeks known to present pulmonary susceptibility were challenged with tobacco smoke in order to induce emphysema. Animals were challenged with the smoke of 3 non-filtered cigarette (1R3; University of Kentucky, Lexington,KY) per chamber (0.25 m$^3$, being able to contain 40 animals), twice a day, 30 minutes each time and 5 days a week using a large-whole body chamber. Nonsmoking, age-matched littermates were used as controls.

Trachea isolation

Animals were sacrificed at T0 (start of experiment), and at T1 (10 weeks). Animals were anesthetized by intraperitoneal injection of a 0.6% of sodium pentobarbital solution at a dosage of 15 ml/kg. A tracheotomy was realized and the trachea canulated.
Then, the trachea was isolated, by cutting it at the larynx and bronchial tubes junction level. Trachea was then rinced with PBS buffer and pooled with other isolated tracheaes in an eppendorf tube containing 1.5ml of trizol. The tubes were placed at a temperature of -80°C up to RNA extraction.

Quantification of CD38 expression levels

Total RNA was then extracted and the cDNA was synthesised using methods similar to the one described in Example 1. CD38 expression levels were then quantified using real-time PCR essentially as described in Example 1 with the exception that the amplification primers were designed on the mouse CD38 (SEQ 5 and SEQ 6). The same parameters were measured (Ct) or computed (µCt, relative gene expression level $2^{(\mu Ct-Ct)}$, etc).

Table II.

| Relative CD38 expression in trachea of non-smoking and smoking mice assessed using real-time RT-PCR. | | | |
|---|---|---|---|
| | T0 | T1 | Mean (µCt) |
| Ct | 30.8 | 29.8 | 30.3 |
| $2^{(\mu Ct-Ct)}$ | 0.7 | 1.4 | |
| N(T1)/N(T0) | 2 | | |
| Results : CD38 expression was shown to increase in trachea macrophages (2x) of mice that were smoking during 10 weeks. | | | |

Example 3 : Detection of CD38 polynucleotide expression levels in an animal model of acute inflammation

LPS-stimulated mice model

Male 6-week old C57b1/6 mice were exposed in a plexiglass container to 100 µg/ml lipopolysaccharide or LPS (in a 0.9% pyrogen-free physiological sodium chloride solution) aerosol for 1 hour, delivered using the SPAG-2 series 6000 nebuliser system (ICN) with a flow rate of 8 liters per minute at a pressure of 26 ± 2 psi. Non-exposed, age-matched littermates were used as controls.

Broncho-alveolar lavages

Animals (control and stimulated groups) were then sacrificed 72h after the end of nebulisation to recover cells from broncho-alveolar lavage fluids essentially as follows. Animals were anesthetized by intra-peritoneal injection of a 0.6% of sodium pentobarbital solution at a dosage of 15 ml/kg. A tracheotomy was realized and the trachea canulated. Then, using a syringe, the lungs were rinsed 6 times with 0.3 ml with a sterile PBS solution PBS without calcium and magnesium), containing 2mM EDTA, warmed at 37°C and the fluid recovered by gentle aspiration. The cell suspension corresponding to 2 mice was collected into conic tubes (Falcon 352096) placed in ice.

Total RNA extraction and cDNA synthesis

Collected cells were centrifuged at 4°C. They were denaturated with Trizol (Life Technologies) in order to allow the isolation of total RNA and the cDNA was synthesised using methods similar to the one described in Example 1.

Quantification of CD38 expression levels

CD38 expression levels were then quantified using real-time PCR essentially as described in Example 1 with the exception that the amplification primers were designed on the mouse CD38 (SEQ 5 and SEQ 6). The same parameters were measured (Ct) or computed ($\mu$Ct, relative gene expression level $2^{(\mu Ct-Ct)}$, etc).

Table III.

| Relative CD38 expression in broncho-alveolar lavages of control and LPS-stimulated mice assessed using real-time RT-PCR. | | | |
|---|---|---|---|
| | control | LPS | Mean ($\mu$Ct) |
| Ct | 32.7 | 31.3 | 32.0 |
| $2^{(\mu Ct-Ct)}$ | 0.6 | 1.6 | |
| N(LPS)/N(control) | 2.7 | | |

Results:CD38 expression was shown to increase in BAL macrophages (2,7X) in mice after stimulation with LPS during 72h.

Example 4 : macrophages derived human blood monocytes protocol

PBMC isolation from fresh blood

Red cells from fresh blood were sedimented in Dextran 4% (Sigma) (v/v).
After Incubation for 40 min at 37°C in 5% $CO_2$, PBMC were isolated on density gradient Ficoll-Paque-Plus (*Pharmacia Biotech)* and centrifugated at 1065g for 30 min at room temperature. The second layer containing PBMCs was recovered and centrifuged at 800g for 15 min at room temperature.
The pellet was resuspended in Hanks' balanced salt medium without $Ca^{2+}$ and $Mg^{2+}$, Centrifuged (450g, 15 min, 4°C) and resuspended in Hank's without $Ca^{2+}$ and $Mg^{2+}$. Cells were counted by Counter Z2 *(Coultronics)* and identified by May-Grünwald-Giemsa staining.
Cells were centrifuged (800g, 5 min, 4°C) and resuspended at $1.65 \times 10^6$ cells/ml in RPMI 1640 without serum and with streptomycin 100$\mu$g/ml /penicillin 100 units/ml.

Monocyte isolation by adherence

Cells were seeded at $2.5 \times 10^7$ cells/dish. Adherence takes place for 1h at 37°C in 5% $CO_2$. Non-adherent lymphocytes were removed.
After two washes with PBS without $Ca^{2+}$ and $Mg^{2+}$, cells were resuspended in RPMI 1640 supplemented with 5% AB human serum and streptomycin 100$\mu$g/ml /penicillin 100 units/ml.

Differentiation into macrophages by culture

Monocyte differentiation into "macrophage-like" cells was obtained after 3 days of culture at 37°C in 5% $CO_2$

Stimulation of macrophages by LPS

10µg/ml LPS (E.Coli O55:B5) was added in RPMI 1640 without serum. The incubation took place during 8h at 37°C in 5% $CO_2$.
Cells were then recovered by scraping in 1 ml of Trizol (Sigma) on ice for RNA isolation and gene expression profile by RT-PCR was determined essentially as described in example 1 with the exception that the amplification primers were designed on the mouse CD38 (sequences 5 and 6).

Table IV.

| Relative CD38 expression in macrophages derived from human-blood monocytes assessed using real-time RT-PCR. | | | | | | | |
|---|---|---|---|---|---|---|---|
| | 3 days | 3 days, LPS stimulation | 7 days | 7 days, LPS stimulation | 8 days | 8 days, LPS stimulation | Mean (Ct) |
| Ct | 24.0 | 20.1 | 25.4 | 21.1 | 25.3 | 21.9 | 23.2 |
| $2^{(\mu Ct-Ct)}$ | 0.5 | 8.5 | 0.2 | 4.3 | 0.2 | 2.4 | |
| N (LPS stimulation)/ N | 17 | | 21.5 | | 12 | | |
| Results : CD38 expression was shown to increase in macrophages derived from human blood monocytes after 8h LPS stimulation (17x after 3 days, 21.5x after 7 days, 12x after 8 days) | | | | | | | |

The contents of all patents, patent applications, published articles, books, reference manuals and abstracts cited herein are hereby incorporated by reference in their entirety to more fully describe the state of the art to which the invention pertains.
As various changes can be made in the above-described subject matter without departing from the scope and spirit of the present invention, it is intended that all subject matter contained in the above description, or defined in the appended claims, be interpreted as descriptive and illustrative of the present invention. Many modifications and variations of the present invention are possible in light of the above teachings.

**Claims**

1. Use of macrophagic CD38 as a biomarker for COPD.

2. A method of determining whether an individual suffers from COPD, wherein said method comprises the steps of

   a) quantifying the macrophagic level of CD38 expression and/or activity in a biological sample recovered from said individual;

   b) determining whether said macrophagic level of CD38 expression and/or activity is elevated in said individual compared to the macrophagic level of CD38 expression and/or activity in a control sample; and

   wherein said elevated level of macrophagic CD38 expression and/or activity is taken as a sign of the presence of COPD.

3. A method of determining whether an individual who already suffers from COPD is susceptible to move into a more advanced stage of COPD, wherein said method comprises the steps of

   a) quantifying the macrophagic level of CD38 expression and/or activity in macrophages of a biological sample recovered from said individual;
   b) determining whether said macrophagic level of CD38 expression and/or activity in said individual is similar to the macrophagic level of CD38 expression and/or activity of an individual suffering from a more advanced stage of COPD; and

   wherein said similar macrophagic level of CD38 expression and/or activity is taken as a sign of the evolution for said individual towards said more advanced stage of COPD.

4. A method of monitoring an individual suffering from COPD, wherein said method comprises the steps of

   a) quantifying the macrophagic level of CD38 expression and/or activity in different samples recovered from the said individual at different time points; and
   b) comparing the macrophagic level of CD38 expression and/or activity in said different samples.

5. A method of assessing the efficacy of a treatment for an individual suffering from COPD and submitted to said treatment, wherein said method comprises the steps of

a) quantifying the macrophagic level of CD38 expression and/or activity in a biological sample recovered from said individual; and
b) determining whether said macrophagic level of CD38 expression and/or activity is modulated in said biological sample compared to the macrophagic level of CD38 expression and/or activity in a control sample.

6.  The method of any one of claims 2 to 5, wherein said quantifying CD38 expression in step a) comprises quantifying CD38 polynucleotides or quantifying CD38 polypeptides present in said biological sample.

7.  The method of any one of claims 2 to 5, wherein step a) comprises quantifying CD38 activity in said biological sample.

8.  A method according to any of claims 2 to 5 wherein said macrophagic CD38 expression and/or activity in said biological sample is elevated from at least 1.7 fold compared to said macrophagic CD38 expression and/or activity in said control sample.

9.  A method according to claims 2-8 wherein said macrophages are recruited macrophages

10. A diagnostic kit for detecting COPD comprising reagents to determine the level of CD38 expression and/or activity.

**European Patent Office**

**EUROPEAN SEARCH REPORT**

Application Number

EP 03 29 2218

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 93 01273 A (UNIV CALIFORNIA ;RUSH PRESBYTERIAN (US)) 21 January 1993 (1993-01-21) * page 8, line 35 - page 9, line 17; claim 13 * | 10 | C12Q1/68 G01N33/68 C07K14/705 |
| X | WO 99 28486 A (UNIV DUKE) 10 June 1999 (1999-06-10) * page 15, line 22 - page 16, line 13; claim 22 * | 10 | |
| Y | WO 02 33377 A (SURROMED INC) 25 April 2002 (2002-04-25) * the whole document * | 1-10 | |
| Y | WO 00 63698 A (SEPPER RUTH ;PRIKK KAIU (EE); RAULO SAARA (FI); TIKANOJA SARI (FI)) 26 October 2000 (2000-10-26) * the whole document * | 1-10 | |
| A | EP 1 211 326 A (GLAXO GROUP LTD) 5 June 2002 (2002-06-05) * claims 1-22 * | 1-10 | TECHNICAL FIELDS SEARCHED (Int.Cl.7) C12Q G01N C07K |
| A | HODGE SANDRA ET AL: "Alveolar macrophages from subjects with chronic obstructive pulmonary disease are deficient in their ability to phagocytose apoptotic airway epithelial cells." IMMUNOLOGY AND CELL BIOLOGY, vol. 81, no. 4, August 2003 (2003-08), pages 289-296, XP001184191 ISSN: 0818-9641 * the whole document * -/-- | 1-10 | |

The present search report has been drawn up for all claims

| Place of search | Date of completion of the search | Examiner |
|---|---|---|
| THE HAGUE | 4 February 2004 | Cornelis, K |

**European Patent Office**

## EUROPEAN SEARCH REPORT

Application Number

EP 03 29 2218

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | GARN HOLGER ET AL: "Shift toward an alternatively activated macrophage response in lungs of NO2-exposed rats." AMERICAN JOURNAL OF RESPIRATORY CELL AND MOLECULAR BIOLOGY, vol. 28, no. 3, March 2003 (2003-03), pages 386-396, XP008026839 ISSN: 1044-1549 (ISSN print) * abstract * | 1-10 | |

TECHNICAL FIELDS SEARCHED (Int.Cl.7)

The present search report has been drawn up for all claims

| Place of search | Date of completion of the search | Examiner |
|---|---|---|
| THE HAGUE | 4 February 2004 | Cornelis, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 514 945 A1**

**ANNEX TO THE EUROPEAN SEARCH REPORT
ON EUROPEAN PATENT APPLICATION NO.**   EP 03 29 2218

04-02-2004

| Patent document cited in search report | | | Publication date | Patent family member(s) | | | Publication date |
|---|---|---|---|---|---|---|---|
| WO 9301273 | A | | 21-01-1993 | US | 5426028 | A | 20-06-1995 |
| | | | | AU | 2338492 | A | 11-02-1993 |
| | | | | WO | 9301273 | A1 | 21-01-1993 |
| | | | | US | 5538856 | A | 23-07-1996 |
| WO 9928486 | A | | 10-06-1999 | AU | 753975 | B2 | 31-10-2002 |
| | | | | AU | 1625199 | A | 16-06-1999 |
| | | | | CA | 2313250 | A1 | 10-06-1999 |
| | | | | EP | 1034288 | A1 | 13-09-2000 |
| | | | | JP | 2001525176 | T | 11-12-2001 |
| | | | | WO | 9928486 | A1 | 10-06-1999 |
| | | | | US | 6537807 | B1 | 25-03-2003 |
| WO 0233377 | A | | 25-04-2002 | AU | 9284601 | A | 29-04-2002 |
| | | | | WO | 0233377 | A2 | 25-04-2002 |
| | | | | US | 2002072484 | A1 | 13-06-2002 |
| WO 0063698 | A | | 26-10-2000 | AU | 4121900 | A | 02-11-2000 |
| | | | | CA | 2370418 | A1 | 26-10-2000 |
| | | | | EP | 1171770 | A1 | 16-01-2002 |
| | | | | WO | 0063698 | A1 | 26-10-2000 |
| | | | | JP | 2002542485 | T | 10-12-2002 |
| EP 1211326 | A | | 05-06-2002 | EP | 1211326 | A2 | 05-06-2002 |
| | | | | US | 2002142294 | A1 | 03-10-2002 |

EPO FORM P0459


<110> PFIZER

<120> CD38 as macrophagic biomarker or target for COPD.

<130> PC025041

<160> 6

<210> 1
<211> 18
<212> ADN
<213> artificial sequence

<223> Synthetic oligonucleotide 1

<400> 1
cctgctgccg gctctcta                                              18

<210> 2
<211> 19
<212> ADN
<213> artificial sequence

<223> Synthetic oligonucleotide 2

<400> 2
agcaccacga cgaggatca                                             19

<210> 3

<211> 21

<212> ADN

<213> artificial sequence

<223> Synthetic oligonucleotide 3

<400> 3

agaaacggct accacatcca a                                    21

<210> 4

<211> 28

<212> ADN

<213> artificial sequence

<223> Synthetic oligonucleotide 4

<400> 4

cgttaaagga tttaaagtgg actcattc                             28

<210> 5

<211> 22

<212> ADN

<213> artificial sequence

<223> Synthetic oligonucleotide 5

<400> 5

caaacacctg gcccatcaat at                                   22

<210> 6

<211> 21

<212> ADN

<213> artificial sequence


<223> Synthetic oligonucleotide 6


<400> 6

tgtcctccag ggtgaacatc t                                                     21